# EUROPÄISCHE PATENTSCHRIFT

(11) **EP 3 385 275 B1**
(45) Veröffentlichungstag und Bekanntmachung des Hinweises auf die Patenterteilung: **23.10.2019**
(21) Anmeldenummer: 17165489.0
(22) Anmeldetag: 07.04.2017
(51) Int. Cl.: C07K 14/245, C12N 1/20, C12N 15/52, C12P 13/22

(54) **VERFAHREN ZUR HERSTELLUNG VON AROMATISCHEN L-AMINOSÄUREN UNTER VERWENDUNG VON VERBESSERTEN STÄMMEN DER FAMILIE ENTEROBACTERIACEAE**
METHOD FOR PRODUCING AROMATIC L-AMINO ACIDS USING IMPROVED STRAINS OF THE ENTEROBACTERIACEAE FAMILY
PROCÉDÉ DE FABRICATION D'AMINOACIDES L AROMATIQUES À L'AIDE DE TRONCS AMÉLIORÉS DE LA FAMILLE DES ENTEROBACTERIACEAE

(43) Veröffentlichungstag der Anmeldung: 10.10.2018
(73) Patentinhaber: Evonik Degussa GmbH, 45128 Essen (DE)
(72) Erfinder: GERTH, Caroline, 33813 Oerlinghausen (DE); RIEPING, Mechthild, 33619 Bielefeld (DE); BATHE, Brigitte, 33154 Salzkotten (DE)
(74) Vertreter: Evonik Patent Association

(56) Entgegenhaltungen:
- DE-A1-102007 051 024
- US-A- 5 939 295
- JIABEI LIN ET AL: "Examination of the dynamic assembly equilibrium for E . coli ClpB : ClpB Assembly", PROTEINS: STRUCTURE, FUNCTION, AND BIOINFORMATICS, Bd. 83, Nr. 11, 1. November 2015 (2015-11-01), Seiten 2008-2024, XP055380012, US ISSN: 0887-3585, DOI: 10.1002/prot.24914

### SEQUENCE LISTING

<110> Evonik Degussa GmbH
<120> Verfahren zur Herstellung von L-Tryptophan unter Verwendung von verbesserten Stämmen der Familie Enterobacteriaceae
<130> 201600334
<160> 14
<170> PatentIn version 3.5
<210> 1
   <211> 2574
   <212> DNA
   <213> Escherichia coli
<220>
   <221> CDS
   <222> (1)..(2574)
   <223> coding sequence clpB
<400> 1
   atg cgt ctg gat cgt ctt act aat aaa ttc cag ctt gct ctt gcc gat      48
   Met Arg Leu Asp Arg Leu Thr Asn Lys Phe Gln Leu Ala Leu Ala Asp
   1               5                   10                  15
   gcc caa tca ctt gca ctc ggg cac gac aac caa ttt atc gaa cca ctt      96
   Ala Gln Ser Leu Ala Leu Gly His Asp Asn Gln Phe Ile Glu Pro Leu
               20                  25                  30
   cat tta atg agc gcc ctg ctg aat cag gaa ggg ggt tcg gtt agt cct      144
   His Leu Met Ser Ala Leu Leu Asn Gln Glu Gly Gly Ser Val Ser Pro
           35                  40                  45
   tta tta aca tcc gct ggc ata aat gct ggc cag ttg cgc aca gat atc      192
   Leu Leu Thr Ser Ala Gly Ile Asn Ala Gly Gln Leu Arg Thr Asp Ile
       50                  55                  60
   aat cag gca tta aat cgt tta ccg cag gtt gaa ggt act ggt ggt gat      240
   Asn Gln Ala Leu Asn Arg Leu Pro Gln Val Glu Gly Thr Gly Gly Asp
   65                  70                  75                  80
   gtc cag cca tca cag gat ctg gtg cgc gtt ctt aat ctt tgc gac aag      288
   Val Gln Pro Ser Gln Asp Leu Val Arg Val Leu Asn Leu Cys Asp Lys
                   85                  90                  95
   ctg gcg caa aaa cgt ggt gat aac ttt atc tcg tca gaa ctg ttc gtt      336
   Leu Ala Gln Lys Arg Gly Asp Asn Phe Ile Ser Ser Glu Leu Phe Val
               100                 105                 110
   ctg gcg gca ctt gag tct cgc ggc acg ctg gcc gac atc ctg aaa gca      384
   Leu Ala Ala Leu Glu Ser Arg Gly Thr Leu Ala Asp Ile Leu Lys Ala
           115                 120                 125
   gca ggg gcg acc acc gcc aac att act caa gcg att gaa caa atg cgt      432
   Ala Gly Ala Thr Thr Ala Asn Ile Thr Gln Ala Ile Glu Gln Met Arg
       130                 135                 140
   gga ggt gaa agc gtg aac gat caa ggt gct gaa gac caa cgt cag gct      480
   Gly Gly Glu Ser Val Asn Asp Gln Gly Ala Glu Asp Gln Arg Gln Ala
   145                 150                 155                 160
   ttg aaa aaa tat acc atc gac ctt acc gaa cga gcc gaa cag ggc aaa        528
   Leu Lys Lys Tyr Thr Ile Asp Leu Thr Glu Arg Ala Glu Gln Gly Lys
                   165                 170                 175
   ctc gat ccg gtg att ggt cgt gat gaa gaa att cgc cgt acc att cag        576
   Leu Asp Pro Val Ile Gly Arg Asp Glu Glu Ile Arg Arg Thr Ile Gln
               180                 185                 190
   gtg ctg caa cgt cgt act aaa aat aac ccg gta ctg att ggt gaa ccc        624
   Val Leu Gln Arg Arg Thr Lys Asn Asn Pro Val Leu Ile Gly Glu Pro
           195                 200                 205
   ggc gtc ggt aaa act gcc atc gtt gaa ggt ctg gcg cag cgt att atc        672
   Gly Val Gly Lys Thr Ala Ile Val Glu Gly Leu Ala Gln Arg Ile Ile
       210                 215                 220
   aac ggc gaa gtg ccg gaa ggg ttg aaa ggc cgc cgg gta ctg gcg ctg        720
   Asn Gly Glu Val Pro Glu Gly Leu Lys Gly Arg Arg Val Leu Ala Leu
   225                 230                 235                 240
   gat atg ggc gcg ctg gtg gct ggg gcg aaa tat cgc ggt gag ttt gaa        768
   Asp Met Gly Ala Leu Val Ala Gly Ala Lys Tyr Arg Gly Glu Phe Glu
                   245                 250                 255
   gaa cgt tta aaa ggc gtg ctt aac gat ctt gcc aaa cag gaa ggc aac        816
   Glu Arg Leu Lys Gly Val Leu Asn Asp Leu Ala Lys Gln Glu Gly Asn
               260                 265                 270
   gtc atc cta ttt atc gac gaa tta cat acc atg gtc ggc gcg ggt aaa        864
   Val Ile Leu Phe Ile Asp Glu Leu His Thr Met Val Gly Ala Gly Lys
           275                 280                 285
   gcc gat ggc gca atg gac gcc gga aac atg ctg aaa ccg gcg ctg gcg        912
   Ala Asp Gly Ala Met Asp Ala Gly Asn Met Leu Lys Pro Ala Leu Ala
       290                 295                 300
   cgt ggt gaa ttg cac tgc gta ggt gcc acg acg ctt gac gaa tat cgc        960
   Arg Gly Glu Leu His Cys Val Gly Ala Thr Thr Leu Asp Glu Tyr Arg
   305                 310                 315                 320
   cag tac att gaa aaa gat gct gcg ctg gaa cgt cgt ttc cag aaa gtg       1008
   Gln Tyr Ile Glu Lys Asp Ala Ala Leu Glu Arg Arg Phe Gln Lys Val
                   325                 330                 335
   ttt gtt gcc gag cct tct gtt gaa gat acc att gcg att ctg cgt ggc       1056
   Phe Val Ala Glu Pro Ser Val Glu Asp Thr Ile Ala Ile Leu Arg Gly
               340                 345                 350
   ctg aaa gaa cgt tac gaa ttg cac cac cat gtg caa att act gac ccg       1104
   Leu Lys Glu Arg Tyr Glu Leu His His His Val Gln Ile Thr Asp Pro
           355                 360                 365
   gca att gtt gca gcg gcg acg ttg tct cat cgc tac att gct gac cgt       1152
   Ala Ile Val Ala Ala Ala Thr Leu Ser His Arg Tyr Ile Ala Asp Arg
       370                 375                 380
   cag ctg ccg gat aaa gcc atc gac ctg atc gat gaa gca gca tcc agc       1200
   Gln Leu Pro Asp Lys Ala Ile Asp Leu Ile Asp Glu Ala Ala Ser Ser
   385                 390                 395                 400
   att cgt atg cag att gac tca aaa cca gaa gaa ctc gac cga ctc gat       1248
   Ile Arg Met Gln Ile Asp Ser Lys Pro Glu Glu Leu Asp Arg Leu Asp
                   405                 410                 415
   cgt cgt atc atc cag ctc aaa ctg gaa caa cag gcg tta atg aaa gag      1296
   Arg Arg Ile Ile Gln Leu Lys Leu Glu Gln Gln Ala Leu Met Lys Glu
               420                 425                 430
   tct gat gaa gcc agt aaa aaa cgt ctg gat atg ctc aac gaa gaa ctg      1344
   Ser Asp Glu Ala Ser Lys Lys Arg Leu Asp Met Leu Asn Glu Glu Leu
           435                 440                 445
   agc gac aaa gaa cgt cag tac tcc gag tta gaa gaa gag tgg aaa gca      1392
   Ser Asp Lys Glu Arg Gln Tyr Ser Glu Leu Glu Glu Glu Trp Lys Ala
       450                 455                 460
   gag aag gca tcg ctt tct ggt acg cag acc att aaa gcg gaa ctg gaa      1440
   Glu Lys Ala Ser Leu Ser Gly Thr Gln Thr Ile Lys Ala Glu Leu Glu
   465                 470                 475                 480
   cag gcg aaa atc gct att gaa cag gct cgc cgt gtg ggg gac ctg gcg      1488
   Gln Ala Lys Ile Ala Ile Glu Gln Ala Arg Arg Val Gly Asp Leu Ala
                   485                 490                 495
   cgg atg tct gaa ctg caa tac ggc aaa atc ccg gaa ctg gaa aag caa      1536
   Arg Met Ser Glu Leu Gln Tyr Gly Lys Ile Pro Glu Leu Glu Lys Gln
               500                 505                 510
   ctg gaa gcc gca acg cag ctc gaa ggc aaa act atg cgt ctg ttg cgt      1584
   Leu Glu Ala Ala Thr Gln Leu Glu Gly Lys Thr Met Arg Leu Leu Arg
           515                 520                 525
   aat aaa gtg acc gac gcc gaa att gct gaa gtg ctg gcg cgt tgg acg      1632
   Asn Lys Val Thr Asp Ala Glu Ile Ala Glu Val Leu Ala Arg Trp Thr
       530                 535                 540
   ggg att ccg gtt tct cgc atg atg gaa agc gag cgc gaa aaa ctg ctg      1680
   Gly Ile Pro Val Ser Arg Met Met Glu Ser Glu Arg Glu Lys Leu Leu
   545                 550                 555                 560
   cgt atg gag caa gaa ctg cac cat cgc gta att ggt cag aac gaa gcg      1728
   Arg Met Glu Gln Glu Leu His His Arg Val Ile Gly Gln Asn Glu Ala
                   565                 570                 575
   gtt gat gcg gta tct aac gct att cgt cgt agc cgt gcg ggg ctg gcg      1776
   Val Asp Ala Val Ser Asn Ala Ile Arg Arg Ser Arg Ala Gly Leu Ala
               580                 585                 590
   gat cca aat cgc ccg att ggt tca ttc ctg ttc ctc ggc cca act ggt      1824
   Asp Pro Asn Arg Pro Ile Gly Ser Phe Leu Phe Leu Gly Pro Thr Gly
           595                 600                 605
   gtg ggg aaa aca gag ctt tgt aag gcg ctg gcg aac ttt atg ttt gat      1872
   Val Gly Lys Thr Glu Leu Cys Lys Ala Leu Ala Asn Phe Met Phe Asp
       610                 615                 620
   agc gac gag gcg atg gtc cgt atc gat atg tcc gag ttt atg gag aaa      1920
   Ser Asp Glu Ala Met Val Arg Ile Asp Met Ser Glu Phe Met Glu Lys
   625                 630                 635                 640
   cac tcg gtg tct cgt ttg gtt ggt gcg cct ccg gga tat gtc ggt tat      1968
   His Ser Val Ser Arg Leu Val Gly Ala Pro Pro Gly Tyr Val Gly Tyr
                   645                 650                 655
   gaa gaa ggt ggc tac ctg acc gaa gcg gtg cgt cgt cgt ccg tat tcc      2016
   Glu Glu Gly Gly Tyr Leu Thr Glu Ala Val Arg Arg Arg Pro Tyr Ser
               660                 665                 670
   gtc atc ctg ctg gat gaa gtg gaa aaa gcg cat ccg gat gtc ttc aac      2064
   Val Ile Leu Leu Asp Glu Val Glu Lys Ala His Pro Asp Val Phe Asn
           675                 680                 685
   att ctg ttg cag gta ctg gat gat ggg cgt ctg act gac ggg caa ggg      2112
   Ile Leu Leu Gln Val Leu Asp Asp Gly Arg Leu Thr Asp Gly Gln Gly
       690                 695                 700
   aga acg gtc gac ttc cgt aat acg gtc gtc att atg acc tct aac ctc      2160
   Arg Thr Val Asp Phe Arg Asn Thr Val Val Ile Met Thr Ser Asn Leu
   705                 710                 715                 720
   ggt tcc gat ctg att cag gaa cgc ttc ggt gaa ctg gat tat gcg cac      2208
   Gly Ser Asp Leu Ile Gln Glu Arg Phe Gly Glu Leu Asp Tyr Ala His
                   725                 730                 735
   atg aaa gag ctg gtg ctc ggt gtg gta agc cat aac ttc cgt ccg gaa      2256
   Met Lys Glu Leu Val Leu Gly Val Val Ser His Asn Phe Arg Pro Glu
               740                 745                 750
   ttc att aac cgt atc gat gaa gtg gtg gtc ttc cat ccg ctg ggt gaa      2304
   Phe Ile Asn Arg Ile Asp Glu Val Val Val Phe His Pro Leu Gly Glu
           755                 760                 765
   cag cac att gcc tcg att gcg cag att cag ttg aaa cgt ctg tac aaa      2352
   Gln His Ile Ala Ser Ile Ala Gln Ile Gln Leu Lys Arg Leu Tyr Lys
       770                 775                 780
   cgt ctg gaa gaa cgt ggt tat gaa atc cac att tct gac gag gcg ctg      2400
   Arg Leu Glu Glu Arg Gly Tyr Glu Ile His Ile Ser Asp Glu Ala Leu
   785                 790                 795                 800
   aaa ctg ctg agc gag aac ggt tac gat ccg gtc tat ggt gca cgt cct      2448
   Lys Leu Leu Ser Glu Asn Gly Tyr Asp Pro Val Tyr Gly Ala Arg Pro
                   805                 810                 815
   ctg aaa cgt gca att cag cag cag atc gaa aac ccg ctg gca cag caa      2496
   Leu Lys Arg Ala Ile Gln Gln Gln Ile Glu Asn Pro Leu Ala Gln Gln
               820                 825                 830
   ata ctg tct ggt gaa ttg gtt ccg ggt aaa gtg att cgc ctg gaa gtt      2544
   Ile Leu Ser Gly Glu Leu Val Pro Gly Lys Val Ile Arg Leu Glu Val
           835                 840                 845
   aat gaa gac cgg att gtc gcc gtc cag taa                              2574
   Asn Glu Asp Arg Ile Val Ala Val Gln
       850                 855
<210> 2
   <211> 857
   <212> PRT
   <213> Escherichia coli
<400> 2
   Met Arg Leu Asp Arg Leu Thr Asn Lys Phe Gln Leu Ala Leu Ala Asp
   1               5                   10                  15
   Ala Gln Ser Leu Ala Leu Gly His Asp Asn Gln Phe Ile Glu Pro Leu
               20                  25                  30
   His Leu Met Ser Ala Leu Leu Asn Gln Glu Gly Gly Ser Val Ser Pro
           35                  40                  45
   Leu Leu Thr Ser Ala Gly Ile Asn Ala Gly Gln Leu Arg Thr Asp Ile
       50                  55                  60
   Asn Gln Ala Leu Asn Arg Leu Pro Gln Val Glu Gly Thr Gly Gly Asp
   65                  70                  75                  80
   Val Gln Pro Ser Gln Asp Leu Val Arg Val Leu Asn Leu Cys Asp Lys
                   85                  90                  95
   Leu Ala Gln Lys Arg Gly Asp Asn Phe Ile Ser Ser Glu Leu Phe Val
               100                 105                 110
   Leu Ala Ala Leu Glu Ser Arg Gly Thr Leu Ala Asp Ile Leu Lys Ala
           115                 120                 125
   Ala Gly Ala Thr Thr Ala Asn Ile Thr Gln Ala Ile Glu Gln Met Arg
       130                 135                 140
   Gly Gly Glu Ser Val Asn Asp Gln Gly Ala Glu Asp Gln Arg Gln Ala
   145                 150                 155                 160
   Leu Lys Lys Tyr Thr Ile Asp Leu Thr Glu Arg Ala Glu Gln Gly Lys
                   165                 170                 175
   Leu Asp Pro Val Ile Gly Arg Asp Glu Glu Ile Arg Arg Thr Ile Gln
               180                 185                 190
   Val Leu Gln Arg Arg Thr Lys Asn Asn Pro Val Leu Ile Gly Glu Pro
           195                 200                 205
   Gly Val Gly Lys Thr Ala Ile Val Glu Gly Leu Ala Gln Arg Ile Ile
       210                 215                 220
   Asn Gly Glu Val Pro Glu Gly Leu Lys Gly Arg Arg Val Leu Ala Leu
   225                 230                 235                 240
   Asp Met Gly Ala Leu Val Ala Gly Ala Lys Tyr Arg Gly Glu Phe Glu
                   245                 250                 255
   Glu Arg Leu Lys Gly Val Leu Asn Asp Leu Ala Lys Gln Glu Gly Asn
               260                 265                 270
   Val Ile Leu Phe Ile Asp Glu Leu His Thr Met Val Gly Ala Gly Lys
   275 280 285
   Ala Asp Gly Ala Met Asp Ala Gly Asn Met Leu Lys Pro Ala Leu Ala
   290 295 300
   Arg Gly Glu Leu His Cys Val Gly Ala Thr Thr Leu Asp Glu Tyr Arg
   305 310 315 320
   Gln Tyr Ile Glu Lys Asp Ala Ala Leu Glu Arg Arg Phe Gln Lys Val
   325 330 335
   Phe Val Ala Glu Pro Ser Val Glu Asp Thr Ile Ala Ile Leu Arg Gly
   340 345 350
   Leu Lys Glu Arg Tyr Glu Leu His His His Val Gln Ile Thr Asp Pro
   355 360 365
   Ala Ile Val Ala Ala Ala Thr Leu Ser His Arg Tyr Ile Ala Asp Arg
   370 375 380
   Gln Leu Pro Asp Lys Ala Ile Asp Leu Ile Asp Glu Ala Ala Ser Ser
   385 390 395 400
   Ile Arg Met Gln Ile Asp Ser Lys Pro Glu Glu Leu Asp Arg Leu Asp
   405 410 415
   Arg Arg Ile Ile Gln Leu Lys Leu Glu Gln Gln Ala Leu Met Lys Glu
   420 425 430
   Ser Asp Glu Ala Ser Lys Lys Arg Leu Asp Met Leu Asn Glu Glu Leu
   435 440 445
   Ser Asp Lys Glu Arg Gln Tyr Ser Glu Leu Glu Glu Glu Trp Lys Ala
   450 455 460
   Glu Lys Ala Ser Leu Ser Gly Thr Gln Thr Ile Lys Ala Glu Leu Glu
   465 470 475 480
   Gln Ala Lys Ile Ala Ile Glu Gln Ala Arg Arg Val Gly Asp Leu Ala
   485 490 495
   Arg Met Ser Glu Leu Gln Tyr Gly Lys Ile Pro Glu Leu Glu Lys Gln
   500 505 510
   Leu Glu Ala Ala Thr Gln Leu Glu Gly Lys Thr Met Arg Leu Leu Arg
               515                 520                 525
   Asn Lys Val Thr Asp Ala Glu Ile Ala Glu Val Leu Ala Arg Trp Thr
       530                 535                 540
   Gly Ile Pro Val Ser Arg Met Met Glu Ser Glu Arg Glu Lys Leu Leu
   545                 550                 555                 560
   Arg Met Glu Gln Glu Leu His His Arg Val Ile Gly Gln Asn Glu Ala
                   565                 570                 575
   Val Asp Ala Val Ser Asn Ala Ile Arg Arg Ser Arg Ala Gly Leu Ala
               580                 585                 590
   Asp Pro Asn Arg Pro Ile Gly Ser Phe Leu Phe Leu Gly Pro Thr Gly
           595                 600                 605
   Val Gly Lys Thr Glu Leu Cys Lys Ala Leu Ala Asn Phe Met Phe Asp
       610                 615                 620
   Ser Asp Glu Ala Met Val Arg Ile Asp Met Ser Glu Phe Met Glu Lys
   625                 630                 635                 640
   His Ser Val Ser Arg Leu Val Gly Ala Pro Pro Gly Tyr Val Gly Tyr
                   645                 650                 655
   Glu Glu Gly Gly Tyr Leu Thr Glu Ala Val Arg Arg Arg Pro Tyr Ser
               660                 665                 670
   Val Ile Leu Leu Asp Glu Val Glu Lys Ala His Pro Asp Val Phe Asn
           675                 680                 685
   Ile Leu Leu Gln Val Leu Asp Asp Gly Arg Leu Thr Asp Gly Gln Gly
       690                 695                 700
   Arg Thr Val Asp Phe Arg Asn Thr Val Val Ile Met Thr Ser Asn Leu
   705                 710                 715                 720
   Gly Ser Asp Leu Ile Gln Glu Arg Phe Gly Glu Leu Asp Tyr Ala His
                   725                 730                 735
   Met Lys Glu Leu Val Leu Gly Val Val Ser His Asn Phe Arg Pro Glu
               740                 745                 750
   Phe Ile Asn Arg Ile Asp Glu Val Val Val Phe His Pro Leu Gly Glu
           755                 760                 765
   Gln His Ile Ala Ser Ile Ala Gln Ile Gln Leu Lys Arg Leu Tyr Lys
       770                 775                 780
   Arg Leu Glu Glu Arg Gly Tyr Glu Ile His Ile Ser Asp Glu Ala Leu
   785                 790                 795                 800
   Lys Leu Leu Ser Glu Asn Gly Tyr Asp Pro Val Tyr Gly Ala Arg Pro
                   805                 810                 815
   Leu Lys Arg Ala Ile Gln Gln Gln Ile Glu Asn Pro Leu Ala Gln Gln
               820                 825                 830
   Ile Leu Ser Gly Glu Leu Val Pro Gly Lys Val Ile Arg Leu Glu Val
           835                 840                 845
   Asn Glu Asp Arg Ile Val Ala Val Gln
       850                 855
<210> 3
   <211> 2574
   <212> DNA
   <213> Shigella flexneri
<220>
   <221> CDS
   <222> (1)..(2574)
   <223> coding sequence clpB
<400> 3
   atg cgt ctg gat cgt ctt act aat aaa ttc cag ctt gct ctt gcc gat        48
   Met Arg Leu Asp Arg Leu Thr Asn Lys Phe Gln Leu Ala Leu Ala Asp
   1               5                   10                  15
   gcc caa tca ctt gca ctc ggg cac gac aac caa ttc atc gaa cca ctt        96
   Ala Gln Ser Leu Ala Leu Gly His Asp Asn Gln Phe Ile Glu Pro Leu
               20                  25                  30
   cat tta atg agc gcc ctg ctg aat cag gaa ggg ggt tcg gtt agt cct       144
   His Leu Met Ser Ala Leu Leu Asn Gln Glu Gly Gly Ser Val Ser Pro
           35                  40                  45
   tta tta aca tcc gct ggc ata aat gct ggc cag ttg cgc aca gat atc       192
   Leu Leu Thr Ser Ala Gly Ile Asn Ala Gly Gln Leu Arg Thr Asp Ile
       50                  55                  60
   aat cag gca tta aat cgt tta ccg cag gtt gaa ggt act ggt ggt gat       240
   Asn Gln Ala Leu Asn Arg Leu Pro Gln Val Glu Gly Thr Gly Gly Asp
   65                  70                  75                  80
   gtc cag cca tca cag gat ctg gtg cgc gtt ctt aat ctt tgc gac aag       288
   Val Gln Pro Ser Gln Asp Leu Val Arg Val Leu Asn Leu Cys Asp Lys
                   85                  90                  95
   ctg gcg caa aaa cgt ggt gat aac ttt atc tcg tca gaa ctg ttc gtt       336
   Leu Ala Gln Lys Arg Gly Asp Asn Phe Ile Ser Ser Glu Leu Phe Val
               100                 105                 110
   ctg gcg gca ctt gag tct cgc ggc acg ctg gcc gac atc ctg aaa gca       384
   Leu Ala Ala Leu Glu Ser Arg Gly Thr Leu Ala Asp Ile Leu Lys Ala
           115                 120                 125
   aca ggg gcg acc acc gcc aac att act caa gcg att gaa caa atg cgt       432
   Thr Gly Ala Thr Thr Ala Asn Ile Thr Gln Ala Ile Glu Gln Met Arg
       130                 135                 140
   gga ggt gaa agc gtg aac gat caa ggt gct gaa gac caa cgt cag gct       480
   Gly Gly Glu Ser Val Asn Asp Gln Gly Ala Glu Asp Gln Arg Gln Ala
   145                 150                 155                 160
   ttg aaa aaa tat acc atc gac ctt acc gaa cga gcc gaa cag ggc aaa       528
   Leu Lys Lys Tyr Thr Ile Asp Leu Thr Glu Arg Ala Glu Gln Gly Lys
                   165                 170                 175
   ctc gat ccg gtg att ggt cgt gat gaa gaa att cgc cgt acc att cag       576
   Leu Asp Pro Val Ile Gly Arg Asp Glu Glu Ile Arg Arg Thr Ile Gln
               180                 185                 190
   gtg ctg caa cgt cgt act aaa aat aac ccg gta ctg att ggt gaa ccc       624
   Val Leu Gln Arg Arg Thr Lys Asn Asn Pro Val Leu Ile Gly Glu Pro
           195                 200                 205
   ggc gtc ggt aaa act gcc atc gtt gaa ggt ctg gcg cag cgt att atc       672
   Gly Val Gly Lys Thr Ala Ile Val Glu Gly Leu Ala Gln Arg Ile Ile
       210                 215                 220
   aac ggc gaa gtg ccg gaa gga ttg aaa ggc cgc cgc gta ctg gcg ctg       720
   Asn Gly Glu Val Pro Glu Gly Leu Lys Gly Arg Arg Val Leu Ala Leu
   225                 230                 235                 240
   gat atg ggc gcg ctg gtg gct ggg gcg aaa tat cgc ggt gag ttt gaa       768
   Asp Met Gly Ala Leu Val Ala Gly Ala Lys Tyr Arg Gly Glu Phe Glu
                   245                 250                 255
   gaa cgt tta aaa ggc gtg ctt aac gat ctt gcc aaa cag gaa ggc aac       816
   Glu Arg Leu Lys Gly Val Leu Asn Asp Leu Ala Lys Gln Glu Gly Asn
               260                 265                 270
   gtc atc ctg ttt atc gac gaa tta cat acc atg gtc ggc gcg ggt aaa       864
   Val Ile Leu Phe Ile Asp Glu Leu His Thr Met Val Gly Ala Gly Lys
           275                 280                 285
   gcc gat ggc gca atg gac gcc gga aac atg ctg aaa ccg gcg ctg gcg       912
   Ala Asp Gly Ala Met Asp Ala Gly Asn Met Leu Lys Pro Ala Leu Ala
       290                 295                 300
   cgt ggt gaa ttg cac tgc gta ggt gcc acg acg ctt gac gaa tat cgc       960
   Arg Gly Glu Leu His Cys Val Gly Ala Thr Thr Leu Asp Glu Tyr Arg
   305                 310                 315                 320
   cag tac atc gaa aaa gat gct gcg ctg gaa cgt cgt ttc cag aaa gtg      1008
   Gln Tyr Ile Glu Lys Asp Ala Ala Leu Glu Arg Arg Phe Gln Lys Val
                   325                 330                 335
   ttt gtt gcc gag cct tct gtt gaa gat acc att gcg att ctg cgt ggc      1056
   Phe Val Ala Glu Pro Ser Val Glu Asp Thr Ile Ala Ile Leu Arg Gly
               340                 345                 350
   ctg aaa gaa cgt tac gaa ttg cac cac cat gtg caa att act gac ccg      1104
   Leu Lys Glu Arg Tyr Glu Leu His His His Val Gln Ile Thr Asp Pro
           355                 360                 365
   gca att gtt gca gca gcg acg ttg tct cat cgc tac att gct gac cgt         1152
   Ala Ile Val Ala Ala Ala Thr Leu Ser His Arg Tyr Ile Ala Asp Arg
       370                 375                 380
   cag ctg ccg gat aaa gcc atc gac ctg atc gat gaa gca gca tcc agc         1200
   Gln Leu Pro Asp Lys Ala Ile Asp Leu Ile Asp Glu Ala Ala Ser Ser
   385                 390                 395                 400
   att cgt atg cag att gac tca aaa cca gaa gaa ctc gac cga ctc gat         1248
   Ile Arg Met Gln Ile Asp Ser Lys Pro Glu Glu Leu Asp Arg Leu Asp
                   405                 410                 415
   cgt cgt atc atc cag ctc aaa ctg gaa caa cag gcg tta atg aaa gag         1296
   Arg Arg Ile Ile Gln Leu Lys Leu Glu Gln Gln Ala Leu Met Lys Glu
               420                 425                 430
   tct gat gaa gcc agt aaa aaa cgc ctg gat atg ctc aac gaa gaa ctg         1344
   Ser Asp Glu Ala Ser Lys Lys Arg Leu Asp Met Leu Asn Glu Glu Leu
           435                 440                 445
   agc gac aaa gaa cgt cag tac tcc gag tta gaa gaa gag tgg aaa gca         1392
   Ser Asp Lys Glu Arg Gln Tyr Ser Glu Leu Glu Glu Glu Trp Lys Ala
       450                 455                 460
   gag aag gca tcg ctt tct ggt acg cag acc att aaa gcg gaa ctg gaa         1440
   Glu Lys Ala Ser Leu Ser Gly Thr Gln Thr Ile Lys Ala Glu Leu Glu
   465                 470                 475                 480
   cag gcg aaa atc gct att gaa cag gct cgc cgt gtg ggg gac ctg gcg         1488
   Gln Ala Lys Ile Ala Ile Glu Gln Ala Arg Arg Val Gly Asp Leu Ala
                   485                 490                 495
   cgg atg tct gaa ctg caa tac ggc aaa atc ccg gaa ctg gaa aag caa         1536
   Arg Met Ser Glu Leu Gln Tyr Gly Lys Ile Pro Glu Leu Glu Lys Gln
               500                 505                 510
   ctg gaa gcc gca acg cag ctc gaa ggc aaa act atg cgt ctg ttg cgt         1584
   Leu Glu Ala Ala Thr Gln Leu Glu Gly Lys Thr Met Arg Leu Leu Arg
           515                 520                 525
   aat aaa gtg acc gat gcc gaa att gct gaa gtg ctg gcg cgt tgg acg         1632
   Asn Lys Val Thr Asp Ala Glu Ile Ala Glu Val Leu Ala Arg Trp Thr
       530                 535                 540
   ggg att ccg gtt tct cgc atg atg gaa agc gag cgc gaa aaa ctg ctg         1680
   Gly Ile Pro Val Ser Arg Met Met Glu Ser Glu Arg Glu Lys Leu Leu
   545                 550                 555                 560
   cgt atg gag caa gaa ctg cac cat cgc gtc att ggt cag aac gaa gcg         1728
   Arg Met Glu Gln Glu Leu His His Arg Val Ile Gly Gln Asn Glu Ala
                   565                 570                 575
   gtt gat gcg gta tct aac gct att cgc cgt agc cgt gcg ggg ttg gcg         1776
   Val Asp Ala Val Ser Asn Ala Ile Arg Arg Ser Arg Ala Gly Leu Ala
               580                 585                 590
   gat cca aat cgc ccg att ggt tca ttc ctg ttc ctc ggc cca acg ggt         1824
   Asp Pro Asn Arg Pro Ile Gly Ser Phe Leu Phe Leu Gly Pro Thr Gly
           595                 600                 605
   gtg ggg aaa act gag ctt tgt aag gcg ctg gcg aac ttt atg ttt gat         1872
   Val Gly Lys Thr Glu Leu Cys Lys Ala Leu Ala Asn Phe Met Phe Asp
       610                 615                 620
   agc gac gag gcg atg gtc cgt atc gat atg tcc gaa ttt atg gag aaa      1920
   Ser Asp Glu Ala Met Val Arg Ile Asp Met Ser Glu Phe Met Glu Lys
   625                 630                 635                 640
   cac tcg gtg tct cgt ctg gtt ggt gcg cct ccg gga tat gtc ggt tat      1968
   His Ser Val Ser Arg Leu Val Gly Ala Pro Pro Gly Tyr Val Gly Tyr
                   645                 650                 655
   gaa gaa ggt ggc tac ctg acc gaa gct gtg cgt cgt cgt ccg tat tcc      2016
   Glu Glu Gly Gly Tyr Leu Thr Glu Ala Val Arg Arg Arg Pro Tyr Ser
               660                 665                 670
   gtc atc ctg ctg gat gaa gtg gaa aaa gcg cat ccg gat gtc ttc aat      2064
   Val Ile Leu Leu Asp Glu Val Glu Lys Ala His Pro Asp Val Phe Asn
           675                 680                 685
   att ctg ttg cag gta ctg gat gat ggg cgt ctg act gac ggg cag ggg      2112
   Ile Leu Leu Gln Val Leu Asp Asp Gly Arg Leu Thr Asp Gly Gln Gly
       690                 695                 700
   aga acg gtc gac ttc cgt aat acg gtt gtc att atg acc tct aac ctc      2160
   Arg Thr Val Asp Phe Arg Asn Thr Val Val Ile Met Thr Ser Asn Leu
   705                 710                 715                 720
   ggt tcc gat ctg att cag gaa cgc ttc ggt gaa ctg gat tat gcg cac      2208
   Gly Ser Asp Leu Ile Gln Glu Arg Phe Gly Glu Leu Asp Tyr Ala His
                   725                 730                 735
   atg aaa gag ctg gtg ctc ggt gtg gtt agc cat aac ttc cgt ccg gaa      2256
   Met Lys Glu Leu Val Leu Gly Val Val Ser His Asn Phe Arg Pro Glu
               740                 745                 750
   ttc att aac cgt att gat gaa gtg gtg gtc ttc cat ccg ctg ggt gaa      2304
   Phe Ile Asn Arg Ile Asp Glu Val Val Val Phe His Pro Leu Gly Glu
           755                 760                 765
   cag cac att gcc tcg att gcg cag att cag ttg aaa cgt ctg tac aaa      2352
   Gln His Ile Ala Ser Ile Ala Gln Ile Gln Leu Lys Arg Leu Tyr Lys
       770                 775                 780
   cgt ctg gaa gaa cgt ggt tat gaa atc cac att tct gac gag gcg ctg      2400
   Arg Leu Glu Glu Arg Gly Tyr Glu Ile His Ile Ser Asp Glu Ala Leu
   785                 790                 795                 800
   aaa ctg ctg agc gag aac ggt tac gat cca gtc tat ggt gca cgt cct      2448
   Lys Leu Leu Ser Glu Asn Gly Tyr Asp Pro Val Tyr Gly Ala Arg Pro
                   805                 810                 815
   ttg aaa cgt gct atc cag cag cag atc gaa aac ccg ctg gca cag caa      2496
   Leu Lys Arg Ala Ile Gln Gln Gln Ile Glu Asn Pro Leu Ala Gln Gln
               820                 825                 830
   ata ctg tct ggt gaa ttg gtt ccg ggt aaa gtg att cgc ctg gaa gtt      2544
   Ile Leu Ser Gly Glu Leu Val Pro Gly Lys Val Ile Arg Leu Glu Val
           835                 840                 845
   aat gaa gac cgg att gtc gct gtc cag taa                              2574
   Asn Glu Asp Arg Ile Val Ala Val Gln
       850                 855
<210> 4
   <211> 857
   <212> PRT
   <213> Shigella flexneri
<400> 4
   Met Arg Leu Asp Arg Leu Thr Asn Lys Phe Gln Leu Ala Leu Ala Asp
   1               5                   10                  15
   Ala Gln Ser Leu Ala Leu Gly His Asp Asn Gln Phe Ile Glu Pro Leu
               20                  25                  30
   His Leu Met Ser Ala Leu Leu Asn Gln Glu Gly Gly Ser Val Ser Pro
           35                  40                  45
   Leu Leu Thr Ser Ala Gly Ile Asn Ala Gly Gln Leu Arg Thr Asp Ile
       50                  55                  60
   Asn Gln Ala Leu Asn Arg Leu Pro Gln Val Glu Gly Thr Gly Gly Asp
   65                  70                  75                  80
   Val Gln Pro Ser Gln Asp Leu Val Arg Val Leu Asn Leu Cys Asp Lys
                   85                  90                  95
   Leu Ala Gln Lys Arg Gly Asp Asn Phe Ile Ser Ser Glu Leu Phe Val
               100                 105                 110
   Leu Ala Ala Leu Glu Ser Arg Gly Thr Leu Ala Asp Ile Leu Lys Ala
           115                 120                 125
   Thr Gly Ala Thr Thr Ala Asn Ile Thr Gln Ala Ile Glu Gln Met Arg
       130                 135                 140
   Gly Gly Glu Ser Val Asn Asp Gln Gly Ala Glu Asp Gln Arg Gln Ala
   145                 150                 155                 160
   Leu Lys Lys Tyr Thr Ile Asp Leu Thr Glu Arg Ala Glu Gln Gly Lys
                   165                 170                 175
   Leu Asp Pro Val Ile Gly Arg Asp Glu Glu Ile Arg Arg Thr Ile Gln
               180                 185                 190
   Val Leu Gln Arg Arg Thr Lys Asn Asn Pro Val Leu Ile Gly Glu Pro
           195                 200                 205
   Gly Val Gly Lys Thr Ala Ile Val Glu Gly Leu Ala Gln Arg Ile Ile
       210                 215                 220
   Asn Gly Glu Val Pro Glu Gly Leu Lys Gly Arg Arg Val Leu Ala Leu
   225                 230                 235                 240
   Asp Met Gly Ala Leu Val Ala Gly Ala Lys Tyr Arg Gly Glu Phe Glu
                   245                 250                 255
   Glu Arg Leu Lys Gly Val Leu Asn Asp Leu Ala Lys Gln Glu Gly Asn
               260                 265                 270
   Val Ile Leu Phe Ile Asp Glu Leu His Thr Met Val Gly Ala Gly Lys
           275                 280                 285
   Ala Asp Gly Ala Met Asp Ala Gly Asn Met Leu Lys Pro Ala Leu Ala
       290                 295                 300
   Arg Gly Glu Leu His Cys Val Gly Ala Thr Thr Leu Asp Glu Tyr Arg
   305                 310                 315                 320
   Gln Tyr Ile Glu Lys Asp Ala Ala Leu Glu Arg Arg Phe Gln Lys Val
                   325                 330                 335
   Phe Val Ala Glu Pro Ser Val Glu Asp Thr Ile Ala Ile Leu Arg Gly
               340                 345                 350
   Leu Lys Glu Arg Tyr Glu Leu His His His Val Gln Ile Thr Asp Pro
           355                 360                 365
   Ala Ile Val Ala Ala Ala Thr Leu Ser His Arg Tyr Ile Ala Asp Arg
       370                 375                 380
   Gln Leu Pro Asp Lys Ala Ile Asp Leu Ile Asp Glu Ala Ala Ser Ser
   385                 390                 395                 400
   Ile Arg Met Gln Ile Asp Ser Lys Pro Glu Glu Leu Asp Arg Leu Asp
                   405                 410                 415
   Arg Arg Ile Ile Gln Leu Lys Leu Glu Gln Gln Ala Leu Met Lys Glu
               420                 425                 430
   Ser Asp Glu Ala Ser Lys Lys Arg Leu Asp Met Leu Asn Glu Glu Leu
           435                 440                 445
   Ser Asp Lys Glu Arg Gln Tyr Ser Glu Leu Glu Glu Glu Trp Lys Ala
       450                 455                 460
   Glu Lys Ala Ser Leu Ser Gly Thr Gln Thr Ile Lys Ala Glu Leu Glu
   465                 470                 475                 480
   Gln Ala Lys Ile Ala Ile Glu Gln Ala Arg Arg Val Gly Asp Leu Ala
                   485                 490                 495
   Arg Met Ser Glu Leu Gln Tyr Gly Lys Ile Pro Glu Leu Glu Lys Gln
               500                 505                 510
   Leu Glu Ala Ala Thr Gln Leu Glu Gly Lys Thr Met Arg Leu Leu Arg
           515                 520                 525
   Asn Lys Val Thr Asp Ala Glu Ile Ala Glu Val Leu Ala Arg Trp Thr
       530                 535                 540
   Gly Ile Pro Val Ser Arg Met Met Glu Ser Glu Arg Glu Lys Leu Leu
   545                 550                 555                 560
   Arg Met Glu Gln Glu Leu His His Arg Val Ile Gly Gln Asn Glu Ala
                   565                 570                 575
   Val Asp Ala Val Ser Asn Ala Ile Arg Arg Ser Arg Ala Gly Leu Ala
               580                 585                 590
   Asp Pro Asn Arg Pro Ile Gly Ser Phe Leu Phe Leu Gly Pro Thr Gly
           595                 600                 605
   Val Gly Lys Thr Glu Leu Cys Lys Ala Leu Ala Asn Phe Met Phe Asp
       610                 615                 620
   Ser Asp Glu Ala Met Val Arg Ile Asp Met Ser Glu Phe Met Glu Lys
   625                 630                 635                 640
   His Ser Val Ser Arg Leu Val Gly Ala Pro Pro Gly Tyr Val Gly Tyr
                   645                 650                 655
   Glu Glu Gly Gly Tyr Leu Thr Glu Ala Val Arg Arg Arg Pro Tyr Ser
               660                 665                 670
   Val Ile Leu Leu Asp Glu Val Glu Lys Ala His Pro Asp Val Phe Asn
           675                 680                 685
   Ile Leu Leu Gln Val Leu Asp Asp Gly Arg Leu Thr Asp Gly Gln Gly
       690                 695                 700
   Arg Thr Val Asp Phe Arg Asn Thr Val Val Ile Met Thr Ser Asn Leu
   705                 710                 715                 720
   Gly Ser Asp Leu Ile Gln Glu Arg Phe Gly Glu Leu Asp Tyr Ala His
                   725                 730                 735
   Met Lys Glu Leu Val Leu Gly Val Val Ser His Asn Phe Arg Pro Glu
               740                 745                 750
   Phe Ile Asn Arg Ile Asp Glu Val Val Val Phe His Pro Leu Gly Glu
           755                 760                 765
   Gln His Ile Ala Ser Ile Ala Gln Ile Gln Leu Lys Arg Leu Tyr Lys
       770                 775                 780
   Arg Leu Glu Glu Arg Gly Tyr Glu Ile His Ile Ser Asp Glu Ala Leu
   785                 790                 795                 800
   Lys Leu Leu Ser Glu Asn Gly Tyr Asp Pro Val Tyr Gly Ala Arg Pro
                   805                 810                 815
   Leu Lys Arg Ala Ile Gln Gln Gln Ile Glu Asn Pro Leu Ala Gln Gln
               820                 825                 830
   Ile Leu Ser Gly Glu Leu Val Pro Gly Lys Val Ile Arg Leu Glu Val
           835                 840                 845
   Asn Glu Asp Arg Ile Val Ala Val Gln
       850                 855
<210> 5
   <211> 2574
   <212> DNA
   <213> Salmonella enterica subsp. enterica serovar Typhimurium LT2
<220>
   <221> CDS
   <222> (1)..(2574)
   <223> coding sequence clpB
<400> 5
   atg cgt ctg gat cgt ctt act aac aaa ttc cag ctt gct ctt gcc gat        48
   Met Arg Leu Asp Arg Leu Thr Asn Lys Phe Gln Leu Ala Leu Ala Asp
   1               5                   10                  15
   gcc cag tcg ctc gcg ctg ggg cac gac aac caa ttc atc gaa cct ctt        96
   Ala Gln Ser Leu Ala Leu Gly His Asp Asn Gln Phe Ile Glu Pro Leu
               20                  25                  30
   cat tta atg agc gcc ttg ctg aac cag gaa ggg gga tcg ata cgt cct       144
   His Leu Met Ser Ala Leu Leu Asn Gln Glu Gly Gly Ser Ile Arg Pro
           35                  40                  45
   tta tta acc tcc gcc ggc att aat gct ggc cag ttg cgc acc gct atc       192
   Leu Leu Thr Ser Ala Gly Ile Asn Ala Gly Gln Leu Arg Thr Ala Ile
       50                  55                  60
   gat cag gcg ctg agc cgt tta ccg cag gtg gaa ggc acc ggc ggc gac         240
   Asp Gln Ala Leu Ser Arg Leu Pro Gln Val Glu Gly Thr Gly Gly Asp
   65                  70                  75                  80
   gta cag cct tct tcg gaa ctg gta cgc gta ctg aac ctt tgc gac aag         288
   Val Gln Pro Ser Ser Glu Leu Val Arg Val Leu Asn Leu Cys Asp Lys
                   85                  90                  95
   ctg gcg caa aaa cgg gga gac aat ttt att tcg tca gag ctg ttt gtt         336
   Leu Ala Gln Lys Arg Gly Asp Asn Phe Ile Ser Ser Glu Leu Phe Val
               100                 105                 110
   ctg gcg gcg ctt gag tct cgc ggc acg tta acc gat ctg ctg aaa tca         384
   Leu Ala Ala Leu Glu Ser Arg Gly Thr Leu Thr Asp Leu Leu Lys Ser
           115                 120                 125
   gcc ggt gcg acc acg gcc aat atc act cag gca att gaa cag atg cgc         432
   Ala Gly Ala Thr Thr Ala Asn Ile Thr Gln Ala Ile Glu Gln Met Arg
       130                 135                 140
   gga ggt gaa agc gtg aac gat caa ggg gct gaa gac caa cgt cag gcc         480
   Gly Gly Glu Ser Val Asn Asp Gln Gly Ala Glu Asp Gln Arg Gln Ala
   145                 150                 155                 160
   ttg aaa aaa tat acc gtc gat ctg acc gag cgg gct gaa caa ggc aag         528
   Leu Lys Lys Tyr Thr Val Asp Leu Thr Glu Arg Ala Glu Gln Gly Lys
                   165                 170                 175
   ctt gac ccg gta att ggt cgt gat gaa gaa att cgc cgt acc att cag         576
   Leu Asp Pro Val Ile Gly Arg Asp Glu Glu Ile Arg Arg Thr Ile Gln
               180                 185                 190
   gta ctg caa cgt cgt acc aaa aac aac ccg gtg tta atc ggt gag cca         624
   Val Leu Gln Arg Arg Thr Lys Asn Asn Pro Val Leu Ile Gly Glu Pro
           195                 200                 205
   ggg gtc ggt aaa acg gca att gtt gaa gga ctg gcg cag cgc att att         672
   Gly Val Gly Lys Thr Ala Ile Val Glu Gly Leu Ala Gln Arg Ile Ile
       210                 215                 220
   aac ggt gaa gtc cct gaa ggc tta aaa ggt cgc cgc gta ctg gcg ctg         720
   Asn Gly Glu Val Pro Glu Gly Leu Lys Gly Arg Arg Val Leu Ala Leu
   225                 230                 235                 240
   gat atg ggc gcg ctg gtg gcg ggg gcg aaa tat cgc ggc gaa ttt gaa         768
   Asp Met Gly Ala Leu Val Ala Gly Ala Lys Tyr Arg Gly Glu Phe Glu
                   245                 250                 255
   gag cgt ctg aaa ggc gtg ctg aac gat ctg gcg aaa cag gaa ggc aac         816
   Glu Arg Leu Lys Gly Val Leu Asn Asp Leu Ala Lys Gln Glu Gly Asn
               260                 265                 270
   gtc atc ctg ttt att gac gag ctg cac acg atg gtt ggc gca ggt aag         864
   Val Ile Leu Phe Ile Asp Glu Leu His Thr Met Val Gly Ala Gly Lys
           275                 280                 285
   gct gat ggc gca atg gac gcc ggg aat atg ctg aaa ccg gcg ctg gcg         912
   Ala Asp Gly Ala Met Asp Ala Gly Asn Met Leu Lys Pro Ala Leu Ala
       290                 295                 300
   cgt ggt gaa ctg cac tgt gtg ggc gcc acc acg ctg gat gaa tac cgt         960
   Arg Gly Glu Leu His Cys Val Gly Ala Thr Thr Leu Asp Glu Tyr Arg
   305                 310                 315                 320
   cag tac att gaa aaa gac gcg gcg ctg gaa cgt cgt ttt caa aaa gtg        1008
   Gln Tyr Ile Glu Lys Asp Ala Ala Leu Glu Arg Arg Phe Gln Lys Val
                   325                 330                 335
   ttt gtc gca gaa cct tca gtg gaa gac acc atc gct att ctg cgt ggc        1056
   Phe Val Ala Glu Pro Ser Val Glu Asp Thr Ile Ala Ile Leu Arg Gly
               340                 345                 350
   ctg aaa gaa cgt tac gag ctg cac cac cat gtg cag atc act gac ccg        1104
   Leu Lys Glu Arg Tyr Glu Leu His His His Val Gln Ile Thr Asp Pro
           355                 360                 365
   gcg att gtg gcg gcg gca aca tta tct cac cgc tat atc gcc gac cgt        1152
   Ala Ile Val Ala Ala Ala Thr Leu Ser His Arg Tyr Ile Ala Asp Arg
       370                 375                 380
   cag ttg ccg gat aaa gct atc gac ctc atc gat gaa gct gca tcc agc        1200
   Gln Leu Pro Asp Lys Ala Ile Asp Leu Ile Asp Glu Ala Ala Ser Ser
   385                 390                 395                 400
   att cgt atg cag att gac tct aag ccg gag gag ctg gac aga ctc gac        1248
   Ile Arg Met Gln Ile Asp Ser Lys Pro Glu Glu Leu Asp Arg Leu Asp
                   405                 410                 415
   cgc cgc att att cag ctc aaa ctg gaa cag cag gcg ttg atg aaa gag        1296
   Arg Arg Ile Ile Gln Leu Lys Leu Glu Gln Gln Ala Leu Met Lys Glu
               420                 425                 430
   tct gac gag gcg agt aaa aaa cgt ctc gat atg ctc aac gaa gaa ctg        1344
   Ser Asp Glu Ala Ser Lys Lys Arg Leu Asp Met Leu Asn Glu Glu Leu
           435                 440                 445
   gac gac aaa gag cgc cag tat tct gag ctg gaa gaa gag tgg aaa gcg        1392
   Asp Asp Lys Glu Arg Gln Tyr Ser Glu Leu Glu Glu Glu Trp Lys Ala
       450                 455                 460
   gaa aaa gcg tcg ctc tct ggt acg caa act att aaa gcg gag ctg gag        1440
   Glu Lys Ala Ser Leu Ser Gly Thr Gln Thr Ile Lys Ala Glu Leu Glu
   465                 470                 475                 480
   cag gcg aag att gcc atc gag cag gcg cgt cgc gtt ggc gac ctg gcg        1488
   Gln Ala Lys Ile Ala Ile Glu Gln Ala Arg Arg Val Gly Asp Leu Ala
                   485                 490                 495
   cga atg tct gaa ctg cag tac ggc aaa att ccg gag ctg gaa aaa cag        1536
   Arg Met Ser Glu Leu Gln Tyr Gly Lys Ile Pro Glu Leu Glu Lys Gln
               500                 505                 510
   ctg gaa gcc gcg acc cag tcg gaa ggt aaa acc atg cgt ctg tta cgt        1584
   Leu Glu Ala Ala Thr Gln Ser Glu Gly Lys Thr Met Arg Leu Leu Arg
           515                 520                 525
   aac aaa gta acg gat gcg gaa att gcc gaa gtg cta gcg cgc tgg acc        1632
   Asn Lys Val Thr Asp Ala Glu Ile Ala Glu Val Leu Ala Arg Trp Thr
       530                 535                 540
   ggt att ccg gtt tcc aga atg ctg gaa ggc gaa cgt gaa aaa ctg ttg        1680
   Gly Ile Pro Val Ser Arg Met Leu Glu Gly Glu Arg Glu Lys Leu Leu
   545                 550                 555                 560
   cgt atg gaa caa gag tta cac agc cgt gtg atc ggg cag aat gaa gcc        1728
   Arg Met Glu Gln Glu Leu His Ser Arg Val Ile Gly Gln Asn Glu Ala
                   565                 570                 575
   gtt gaa gca gta tcg aac gcc ata cgt cgt agc cgt gct ggg ctg tcc       1776
   Val Glu Ala Val Ser Asn Ala Ile Arg Arg Ser Arg Ala Gly Leu Ser
               580                 585                 590
   gat ccg aac cgt ccg att ggt tcc ttc ctg ttc ctg ggg ccg acg ggg       1824
   Asp Pro Asn Arg Pro Ile Gly Ser Phe Leu Phe Leu Gly Pro Thr Gly
           595                 600                 605
   gtc ggt aaa acc gaa ctg tgt aaa gca ctg gct aac ttt atg ttc gac       1872
   Val Gly Lys Thr Glu Leu Cys Lys Ala Leu Ala Asn Phe Met Phe Asp
       610                 615                 620
   agc gac gac gcg atg gtg cgt atc gac atg tct gag ttt atg gag aaa       1920
   Ser Asp Asp Ala Met Val Arg Ile Asp Met Ser Glu Phe Met Glu Lys
   625                 630                 635                 640
   cac tcc gtg tct cgt ttg gtc ggg gcg cct ccg gga tat gtc ggc tat       1968
   His Ser Val Ser Arg Leu Val Gly Ala Pro Pro Gly Tyr Val Gly Tyr
                   645                 650                 655
   gaa gaa ggc ggt tat ttg acg gaa gcg gtg cgc cgt cgc cct tat tcc       2016
   Glu Glu Gly Gly Tyr Leu Thr Glu Ala Val Arg Arg Arg Pro Tyr Ser
               660                 665                 670
   gtc atc ttg ttg gat gaa gtg gaa aaa gcg cat ccg gat gta ttc aac       2064
   Val Ile Leu Leu Asp Glu Val Glu Lys Ala His Pro Asp Val Phe Asn
           675                 680                 685
   att ctg ttg cag gtg ctg gac gac ggt cga ctg act gac ggg cag ggg       2112
   Ile Leu Leu Gln Val Leu Asp Asp Gly Arg Leu Thr Asp Gly Gln Gly
       690                 695                 700
   aga acg gtc gac ttc cgt aat acg gtg gtc att atg act tct aac ctc       2160
   Arg Thr Val Asp Phe Arg Asn Thr Val Val Ile Met Thr Ser Asn Leu
   705                 710                 715                 720
   ggt tcc gat ctc att cag gag cgc ttt ggc gaa ctg gat tac ggt cga       2208
   Gly Ser Asp Leu Ile Gln Glu Arg Phe Gly Glu Leu Asp Tyr Gly Arg
                   725                 730                 735
   atg aaa gag atg gtg ctg ggt gtg gtt agc caa aac ttc cgt ccg gaa       2256
   Met Lys Glu Met Val Leu Gly Val Val Ser Gln Asn Phe Arg Pro Glu
               740                 745                 750
   ttt atc aac cgt att gat gaa gtt gtg gta ttc cat ccg tta ggt gaa       2304
   Phe Ile Asn Arg Ile Asp Glu Val Val Val Phe His Pro Leu Gly Glu
           755                 760                 765
   caa cac atc gct tct att gct cag atc cag ctg cag cgt ctg tac aaa       2352
   Gln His Ile Ala Ser Ile Ala Gln Ile Gln Leu Gln Arg Leu Tyr Lys
       770                 775                 780
   cgt ctg gaa gaa cgt ggt tat gaa atc cat atc tcc gac gag gcg ctg       2400
   Arg Leu Glu Glu Arg Gly Tyr Glu Ile His Ile Ser Asp Glu Ala Leu
   785                 790                 795                 800
   aaa ctg ttg agc gcc aac ggt tac gat ccg gtc tac ggg gcg cgt ccg       2448
   Lys Leu Leu Ser Ala Asn Gly Tyr Asp Pro Val Tyr Gly Ala Arg Pro
                   805                 810                 815
   tta aaa cgt gct att caa cag cag atc gaa aac cca ctg gcg cag caa       2496
   Leu Lys Arg Ala Ile Gln Gln Gln Ile Glu Asn Pro Leu Ala Gln Gln
               820                 825                 830
   atc ctg tct ggt gaa ctg gtt cct ggc aaa gtg att cgc ctt gaa gcc       2544
   Ile Leu Ser Gly Glu Leu Val Pro Gly Lys Val Ile Arg Leu Glu Ala
           835                 840                 845
   aat gat gat cgt att gtg gca gtg cag taa                               2574
   Asn Asp Asp Arg Ile Val Ala Val Gln
       850                 855
<210> 6
   <211> 857
   <212> PRT
   <213> Salmonella enterica subsp. enterica serovar Typhimurium LT2
<400> 6
   Met Arg Leu Asp Arg Leu Thr Asn Lys Phe Gln Leu Ala Leu Ala Asp
   1               5                   10                  15
   Ala Gln Ser Leu Ala Leu Gly His Asp Asn Gln Phe Ile Glu Pro Leu
               20                  25                  30
   His Leu Met Ser Ala Leu Leu Asn Gln Glu Gly Gly Ser Ile Arg Pro
           35                  40                  45
   Leu Leu Thr Ser Ala Gly Ile Asn Ala Gly Gln Leu Arg Thr Ala Ile
       50                  55                  60
   Asp Gln Ala Leu Ser Arg Leu Pro Gln Val Glu Gly Thr Gly Gly Asp
   65                  70                  75                  80
   Val Gln Pro Ser Ser Glu Leu Val Arg Val Leu Asn Leu Cys Asp Lys
                   85                  90                  95
   Leu Ala Gln Lys Arg Gly Asp Asn Phe Ile Ser Ser Glu Leu Phe Val
               100                 105                 110
   Leu Ala Ala Leu Glu Ser Arg Gly Thr Leu Thr Asp Leu Leu Lys Ser
           115                 120                 125
   Ala Gly Ala Thr Thr Ala Asn Ile Thr Gln Ala Ile Glu Gln Met Arg
       130                 135                 140
   Gly Gly Glu Ser Val Asn Asp Gln Gly Ala Glu Asp Gln Arg Gln Ala
   145                 150                 155                 160
   Leu Lys Lys Tyr Thr Val Asp Leu Thr Glu Arg Ala Glu Gln Gly Lys
                   165                 170                 175
   Leu Asp Pro Val Ile Gly Arg Asp Glu Glu Ile Arg Arg Thr Ile Gln
               180                 185                 190
   Val Leu Gln Arg Arg Thr Lys Asn Asn Pro Val Leu Ile Gly Glu Pro
           195                 200                 205
   Gly Val Gly Lys Thr Ala Ile Val Glu Gly Leu Ala Gln Arg Ile Ile
       210                 215                 220
   Asn Gly Glu Val Pro Glu Gly Leu Lys Gly Arg Arg Val Leu Ala Leu
   225                 230                 235                 240
   Asp Met Gly Ala Leu Val Ala Gly Ala Lys Tyr Arg Gly Glu Phe Glu
                   245                 250                 255
   Glu Arg Leu Lys Gly Val Leu Asn Asp Leu Ala Lys Gln Glu Gly Asn
               260                 265                 270
   Val Ile Leu Phe Ile Asp Glu Leu His Thr Met Val Gly Ala Gly Lys
           275                 280                 285
   Ala Asp Gly Ala Met Asp Ala Gly Asn Met Leu Lys Pro Ala Leu Ala
       290                 295                 300
   Arg Gly Glu Leu His Cys Val Gly Ala Thr Thr Leu Asp Glu Tyr Arg
   305                 310                 315                 320
   Gln Tyr Ile Glu Lys Asp Ala Ala Leu Glu Arg Arg Phe Gln Lys Val
                   325                 330                 335
   Phe Val Ala Glu Pro Ser Val Glu Asp Thr Ile Ala Ile Leu Arg Gly
               340                 345                 350
   Leu Lys Glu Arg Tyr Glu Leu His His His Val Gln Ile Thr Asp Pro
           355                 360                 365
   Ala Ile Val Ala Ala Ala Thr Leu Ser His Arg Tyr Ile Ala Asp Arg
       370                 375                 380
   Gln Leu Pro Asp Lys Ala Ile Asp Leu Ile Asp Glu Ala Ala Ser Ser
   385                 390                 395                 400
   Ile Arg Met Gln Ile Asp Ser Lys Pro Glu Glu Leu Asp Arg Leu Asp
                   405                 410                 415
   Arg Arg Ile Ile Gln Leu Lys Leu Glu Gln Gln Ala Leu Met Lys Glu
               420                 425                 430
   Ser Asp Glu Ala Ser Lys Lys Arg Leu Asp Met Leu Asn Glu Glu Leu
   435 440 445
   Asp Asp Lys Glu Arg Gln Tyr Ser Glu Leu Glu Glu Glu Trp Lys Ala
   450 455 460
   Glu Lys Ala Ser Leu Ser Gly Thr Gln Thr Ile Lys Ala Glu Leu Glu
   465 470 475 480
   Gln Ala Lys Ile Ala Ile Glu Gln Ala Arg Arg Val Gly Asp Leu Ala
   485 490 495
   Arg Met Ser Glu Leu Gln Tyr Gly Lys Ile Pro Glu Leu Glu Lys Gln
   500 505 510
   Leu Glu Ala Ala Thr Gln Ser Glu Gly Lys Thr Met Arg Leu Leu Arg
   515 520 525
   Asn Lys Val Thr Asp Ala Glu Ile Ala Glu Val Leu Ala Arg Trp Thr
   530 535 540
   Gly Ile Pro Val Ser Arg Met Leu Glu Gly Glu Arg Glu Lys Leu Leu
   545 550 555 560
   Arg Met Glu Gln Glu Leu His Ser Arg Val Ile Gly Gln Asn Glu Ala
   565 570 575
   Val Glu Ala Val Ser Asn Ala Ile Arg Arg Ser Arg Ala Gly Leu Ser
   580 585 590
   Asp Pro Asn Arg Pro Ile Gly Ser Phe Leu Phe Leu Gly Pro Thr Gly
   595 600 605
   Val Gly Lys Thr Glu Leu Cys Lys Ala Leu Ala Asn Phe Met Phe Asp
   610 615 620
   Ser Asp Asp Ala Met Val Arg Ile Asp Met Ser Glu Phe Met Glu Lys
   625 630 635 640
   His Ser Val Ser Arg Leu Val Gly Ala Pro Pro Gly Tyr Val Gly Tyr
   645 650 655
   Glu Glu Gly Gly Tyr Leu Thr Glu Ala Val Arg Arg Arg Pro Tyr Ser
   660 665 670
   Val Ile Leu Leu Asp Glu Val Glu Lys Ala His Pro Asp Val Phe Asn
   675 680 685
   Ile Leu Leu Gln Val Leu Asp Asp Gly Arg Leu Thr Asp Gly Gln Gly
       690                 695                 700
   Arg Thr Val Asp Phe Arg Asn Thr Val Val Ile Met Thr Ser Asn Leu
   705                 710                 715                 720
   Gly Ser Asp Leu Ile Gln Glu Arg Phe Gly Glu Leu Asp Tyr Gly Arg
                   725                 730                 735
   Met Lys Glu Met Val Leu Gly Val Val Ser Gln Asn Phe Arg Pro Glu
               740                 745                 750
   Phe Ile Asn Arg Ile Asp Glu Val Val Val Phe His Pro Leu Gly Glu
           755                 760                 765
   Gln His Ile Ala Ser Ile Ala Gln Ile Gln Leu Gln Arg Leu Tyr Lys
       770                 775                 780
   Arg Leu Glu Glu Arg Gly Tyr Glu Ile His Ile Ser Asp Glu Ala Leu
   785                 790                 795                 800
   Lys Leu Leu Ser Ala Asn Gly Tyr Asp Pro Val Tyr Gly Ala Arg Pro
                   805                 810                 815
   Leu Lys Arg Ala Ile Gln Gln Gln Ile Glu Asn Pro Leu Ala Gln Gln
               820                 825                 830
   Ile Leu Ser Gly Glu Leu Val Pro Gly Lys Val Ile Arg Leu Glu Ala
           835                 840                 845
   Asn Asp Asp Arg Ile Val Ala Val Gln
       850                 855
<210> 7
   <211> 1073
   <212> DNA
   <213> Escherichia coli
<220>
   <221> CDS
   <222> (538)..(1071)
   <223> partial coding sequence clpBT7I
<400> 7
   taggtagcgg ccgccaatcg agcgggaacg gaagtcgccg ccgctcatgc tggctggcgt      60
   ggactgtgcg caggcgtgct ggaagagacg gtttcctgtt ttgctgataa tccggaaaat     120
   attctcgcct ggttagggcc ggcaattggt ccacgcgcgt tcgaagtggg gggggaggtt     180
   cgcgaggcgt ttatggcagt agacgctaaa gcaagtgcag ctttcattca gcatggtgat      240
   aagtatctgg cggatattta tcagcttgcc cggcagcgtc tggcgaacgt gggtgttgag      300
   caaattttcg gcggcgaccg ttgtacatat acggaaaatg agactttctt ctcttatcgt      360
   cgcgacaaga ccaccggtcg tatggcaagt ttcatttggc tgatataacc taaagaatca      420
   agacgatccg gtacgcgtga ttttcttttc acattaatct ggtcaataac cttgaataat      480
   tgagggatga cctcatttaa tctccagtag caactttgat ccgttatggg aggagtt         537
   atg cgt ctg gat cgt ctt att aat aaa ttc cag ctt gct ctt gcc gat        585
   Met Arg Leu Asp Arg Leu Ile Asn Lys Phe Gln Leu Ala Leu Ala Asp
   1               5                   10                  15
   gcc caa tca ctt gca ctc ggg cac gac aac caa ttt atc gaa cca ctt        633
   Ala Gln Ser Leu Ala Leu Gly His Asp Asn Gln Phe Ile Glu Pro Leu
               20                  25                  30
   cat tta atg agc gcc ctg ctg aat cag gaa ggg ggt tcg gtt agt cct        681
   His Leu Met Ser Ala Leu Leu Asn Gln Glu Gly Gly Ser Val Ser Pro
           35                  40                  45
   tta tta aca tcc gct ggc ata aat gct ggc cag ttg cgc aca gat atc        729
   Leu Leu Thr Ser Ala Gly Ile Asn Ala Gly Gln Leu Arg Thr Asp Ile
       50                  55                  60
   aat cag gca tta aat cgt tta ccg cag gtt gaa ggt act ggt ggt gat        777
   Asn Gln Ala Leu Asn Arg Leu Pro Gln Val Glu Gly Thr Gly Gly Asp
   65                  70                  75                  80
   gtc cag cca tca cag gat ctg gtg cgc gtt ctt aat ctt tgc gac aag        825
   Val Gln Pro Ser Gln Asp Leu Val Arg Val Leu Asn Leu Cys Asp Lys
                   85                  90                  95
   ctg gcg caa aaa cgt ggt gat aac ttt atc tcg tca gaa ctg ttc gtt        873
   Leu Ala Gln Lys Arg Gly Asp Asn Phe Ile Ser Ser Glu Leu Phe Val
               100                 105                 110
   ctg gcg gca ctt gag tct cgc ggc acg ctg gcc gac atc ctg aaa gca        921
   Leu Ala Ala Leu Glu Ser Arg Gly Thr Leu Ala Asp Ile Leu Lys Ala
           115                 120                 125
   gca ggg gcg acc acc gcc aac att act caa gcg att gaa caa atg cgt        969
   Ala Gly Ala Thr Thr Ala Asn Ile Thr Gln Ala Ile Glu Gln Met Arg
       130                 135                 140
   gga ggt gaa agc gtg aac gat caa ggt gct gaa gac caa cgt cag gct       1017
   Gly Gly Glu Ser Val Asn Asp Gln Gly Ala Glu Asp Gln Arg Gln Ala
   145                 150                 155                 160
   ttg aaa aaa tat acc atc gac ctt acc gaa cga gcc gaa cag gcg gcc       1065
   Leu Lys Lys Tyr Thr Ile Asp Leu Thr Glu Arg Ala Glu Gln Ala Ala
                   165                 170                 175
   gct aac tg                                                            1073
   Ala Asn
<210> 8
   <211> 178
   <212> PRT
   <213> Escherichia coli
<400> 8
   Met Arg Leu Asp Arg Leu Ile Asn Lys Phe Gln Leu Ala Leu Ala Asp
   1               5                   10                  15
   Ala Gln Ser Leu Ala Leu Gly His Asp Asn Gln Phe Ile Glu Pro Leu
               20                  25                  30
   His Leu Met Ser Ala Leu Leu Asn Gln Glu Gly Gly Ser Val Ser Pro
           35                  40                  45
   Leu Leu Thr Ser Ala Gly Ile Asn Ala Gly Gln Leu Arg Thr Asp Ile
       50                  55                  60
   Asn Gln Ala Leu Asn Arg Leu Pro Gln Val Glu Gly Thr Gly Gly Asp
   65                  70                  75                  80
   Val Gln Pro Ser Gln Asp Leu Val Arg Val Leu Asn Leu Cys Asp Lys
                   85                  90                  95
   Leu Ala Gln Lys Arg Gly Asp Asn Phe Ile Ser Ser Glu Leu Phe Val
               100                 105                 110
   Leu Ala Ala Leu Glu Ser Arg Gly Thr Leu Ala Asp Ile Leu Lys Ala
           115                 120                 125
   Ala Gly Ala Thr Thr Ala Asn Ile Thr Gln Ala Ile Glu Gln Met Arg
       130                 135                 140
   Gly Gly Glu Ser Val Asn Asp Gln Gly Ala Glu Asp Gln Arg Gln Ala
   145                 150                 155                 160
   Leu Lys Lys Tyr Thr Ile Asp Leu Thr Glu Arg Ala Glu Gln Ala Ala
                   165                 170                 175
   Ala Asn
<210> 9
   <211> 36
   <212> DNA
   <213> Escherichia coli
<220>
   <221> misc_feature
   <222> (1)..(36)
   <223> Primer clpBT7I_lfw
<220>
   <221> misc_feature
   <222> (1)..(6)
   <223> random
<220>
   <221> misc_feature
   <222> (7)..(17)
   <223> Restrictionsite NotI
<400> 9
   cagttagcgg ccgcctgttc ggctcgttcg gtaagg 36
<210> 10
   <211> 46
   <212> DNA
   <213> Escherichia coli
<220>
   <221> misc_feature
   <222> (1)..(46)
   <223> Primer clpBT7I_lrv
<220>
   <221> mutation
   <222> (23)..(23)
   <223> g2732176a (clpBT7I)
<400> 10
   gttatgcgtc tggatcgtct tattaataaa ttccagcttg ctcttg 46
<210> 11
   <211> 46
   <212> DNA
   <213> Escherichia coli
<220>
   <221> misc_feature
   <222> (1)..(46)
   <223> Primer clpBT7I_rfw
<220>
   <221> mutation
   <222> (24)..(24)
   <223> g2732176a (clpBT7I)
<400> 11
   caagagcaag ctggaattta ttaataagac gatccagacg cataac 46
<210> 12
   <211> 36
   <212> DNA
   <213> Escherichia coli
<220>
   <221> misc_feature
   <222> (1)..(36)
   <223> Primer clpBT7I_rrv
<220>
   <221> misc_feature
   <222> (1)..(6)
   <223> random
<220>
   <221> misc_feature
   <222> (7)..(14)
   <223> restriction site NotI
<400> 12
   taggtagcgg ccgccaatcg agcgggaacg gaagtc 36
<210> 13
   <211> 21
   <212> DNA
   <213> Escherichia coli
<220>
   <221> misc_feature
   <222> (1)..(21)
   <223> Primer pKO3-L
<400> 13
   agggcagggt cgttaaatag c 21
<210> 14
   <211> 21
   <212> DNA
   <213> Escherichia coli
<220>
   <221> misc_feature
   <222> (1)..(21)
   <223> Primer pKO3-R
<400> 14
   ttaatgcgcc gctacagggc g 21

## Beschreibung

### Gebiet der Erfindung

Die vorlegende Erfindung betrifft eine Nukleotidsequenz, die für eine Variante des Hitzeschockproteins ClpB kodiert; rekombinante DNA Sequenzen, die diese Nukleotidsequenz enthalten; Mikroorganismen, die die erfindungsgemäße Nukleotidsequenz oder ein erfindungsgemäßes DNA-Fragment enthalten sowie ein Verfahren zur Herstellung von aromatischen L-Aminosäuren in Wege der Fermentation.

### Hintergrund der Erfindung

Aromatische L-Aminosäuren finden in der Humanmedizin und in der pharmazeutischen Industrie, in der Lebensmittelindustrie und in der Tierernährung Anwendung.

Es ist bekannt, dass aromatische L-Aminosäuren durch Fermentation von Stämmen der *Enterobacteriaceae,* insbesondere *Escherichia coli* (*E*. *coli*) und *Serratia marcescens,* hergestellt werden können. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. lonenaustauschchromatographie, oder die intrinsischen Leistungseigenschaften des Mikroorganismus selbst betreffen.

Seit einigen Jahren werden ebenfalls Methoden der rekombinanten DNA-Technik zur gezielten Stammverbesserung L-Aminosäuren produzierender Stämme der Familie *Enterobacteriaceae* eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von *Escherichia coli* und *Salmonella* sind in Neidhardt (ed): Escherichia coli and Salmonella, Cellular and Molecular Biology, 2nd edition, ASM Press, Washington, D.C., USA, (1996) zu finden.

Durch die Komplexität des Biosyntheseweges der aromatischen L-Aminosäuren und wegen dessen Vernetzung mit vielen anderen Stoffwechselwegen in der Zelle ist nicht vorhersagbar, mit welchen genetischen Variationen oder Modifikationen eines Stammes eine verbesserte Produktion der aromatischen L-Aminosäure, bevorzugt von L-Tryptophan, erzielt werden kann.

Es besteht weiter der Bedarf, insbesondere diejenigen Stämme der Familie *Enterobacteriaceae,* die zur Produktion von aromatischen L-Aminosäuren, insbesondere zur Produktion von L-Tryptophan, geeignet sind, weiter zu optimieren und somit verbesserte Verfahren zur fermentativen Herstellung von aromatischen L-Aminosäuren mit Mikroorganismen der Familie *Enterobacteriaceae* bereitzustellen.

Dementsprechend ist es auch Aufgabe der vorliegenden Erfindung, ein Verfahren zur effizienten Herstellung von aromatischen L-Aminosäuren durch Fermentation bereitzustellen. Zu diesem Zweck wurden neue, rekombinante Mikroorganismen aus der Familie der *Enterobacteriaceae* entwickelt.

### Zusammenfassung der Erfindung

Gegenstand der vorliegenden Erfindung ist eine Nukleotidsequenz, die für eine Variante des Hitzeschockproteins ClpB kodiert, und deren Aminosäuresequenz zu mindestens 95 % identisch ist mit SEQ ID NO:2, wobei in der Aminosäuresequenz der L-Threoninrest an Position 7 gegen einen L-Isoleucinrest ersetzt ist.

Weiterhin betrifft die vorliegende Erfindung DNA-Fragmente und Vektoren, die besagte Nukleotidsequenz enthalten; Mikroorganismen, die mit besagter Nukleotidsequenz ausgestattet wurden sowie ein Verfahren zur Herstellung von aromatischen L-Aminosäuren in Wege der Fermentation.

### Detaillierte Beschreibung der Erfindung

Bei den der Erfindung zugrundeliegenden Arbeiten wurde überraschenderweise gefunden, dass der erfindungsgemäße Mikroorganismus, der die erfindungsgemäße Nukleotidsequenz enthält, vermehrt aromatische L-Aminosäuren, insbesondere L-Tryptophan, produziert und diese in der Zelle oder im Medium anreichern.

Die vorliegende Erfindung betrifft eine Nukleotidsequenz, die für eine Variante des Hitzeschockproteins CIpB kodiert, und deren Aminosäuresequenz zu mindestens 95 % identisch ist mit SEQ ID NO:2, wobei in der Aminosäuresequenz der L-Threoninrest an Position 7 gegen einen L-Isoleucinrest ersetzt ist.

Vorzugsweise kodiert die erfindungsgemäße Nukleotidsequenz für Polypeptide, die zu mindestens 96 %, besonders bevorzugt zu mindestens 97 % oder mindestens 98 % und ganz besonders bevorzugt zu mindestens 99 % oder zu 100% identisch sind mit SEQ ID NO:2 und zwingend der L-Threoninrest an Position 7 gegen einen L-Isoleucinrest ersetzt ist.

Ebenso betrifft die Erfindung eine Nukleotidsequenz, die für eine Variante des Hitzeschockproteins CIpB kodiert, deren Aminosäuresequenz die Sequenz von SEQ ID NO: 2 aufweist, einschließlich der Substitution, Deletion, Insertion und/oder Inversion von 1 bis 20 Aminosäuren, vorzugsweise von 1 bis 10 Aminosäuren, wobei zwingend davon der L-Threoninrest an Position 7 gegen einen L-Isoleucinrest ersetzt ist.

Das von besagter Nukleotidsequenz kodierte Polypeptid besitzt vorzugsweise eine Länge entsprechend 857 Aminosäuren.

Weiterhin ist auch eine Nukleotidsequenz, die die zuvor beschriebene Nukleotidsequenz enthält oder die ein Fragment der zuvor beschriebenen Nukleotidsequenz darstellt, Gegenstand der vorliegenden Erfindung. Ein solches Fragment kodiert mindestens für die Positionen 5 bis 820 der Aminosäuresequenz der zuvor beschriebenen Nukleotidsequenz. Besagtes Fragment weist den Austausch des L-Threoninrests an Position 7 gegen einen L-Isoleucinrest auf. Die Funktionalität der Nuleotidsequenz bleibt in besagtem Fragement erhalten.

Die Nukleotidsequenzen der Gene oder offenen Leserahmen (ORF) von *Escherichia coli* gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Das clpB-Gen von *Escherichia coli* wird unter anderem durch folgende Angaben beschrieben:

| | |
|---|---|
| Beschreibung: | Chaperone protein ClpB |
| Alternativer Name: | Hitzeschockprotein F84.1 |
| Referenz: | Kitagawa et al.; Journal of Bacteriology 173(14): 4247 - 4253 (1991) |
| Accession No.: | NC_000913.3, Region 2731600-2734173 |
| Alternativer Genname: | b*2592* |

Das kodierte Polypeptid besitzt eine Länge von 857 Aminosäuren.

Die Nukleotidsequenen von *Shigella, Salmonella, Klebsiella* und *Enterobacter* gehören ebenfalls zum Stand der Technik. Aus den ebenfalls zur Familie *Enterobacteriaceae* gehörenden *Shigella flexneri* und *Salmonella enterica* ist die Nukleotidsequenz für das clpB-Gen ebenso bekannt (Accession No.: NC_004337.2 (REGION: 2731535-2734108) und Accession No.: NC_003197.2 (REGION: komplementär (2802119-2804692)). Weitere Nukleotidsequenzen für das clpB-Gen findet man aus folgenden *Enterobacteriaceae: Shigella boydii* (Accession No.: WP_073691276.1), *Shigella dysenteriae* (Accession No.: AHA66636.1); *Shigella sonnei* (Accession No.: WP_052992191.1), *Klebsiella pneumoniae* (Accession No.: WP_040215337.1), *Enterobacter cloacae* (Accession No.: WP_063941894.1).

Die Nukleotidsequenzen können den Datenbanken des National Center of Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Hinxon, UK) oder der DNA Datenbank von Japan (DDBJ, Mishima, Japan) entnommen werden.

Der besseren Übersichtlichkeit wegen ist die bekannte Sequenz zum clpB-Gen von *Escherichia coli* unter der SEQ ID No.1 dargestellt. Die hiervon codierte Aminosäuresequenz ist unter SEQ ID No.2 dargestellt. Die bekannte Sequenz zum clpB-Gen von *Shigella flexneri ist* unter der SEQ ID No.3 dargestellt. Die hiervon codierte Aminosäuresequenz ist unter SEQ ID No.4 dargestellt. Die bekannte Sequenz zum clpB-Gen von *Salmonella enterica ist* unter der SEQ ID No.5 dargestellt. Die hiervon codierte Aminosäuresequenz ist unter SEQ ID No.6 dargestellt.

Die in den angegebenen Stellen beschriebenen Gene können erfindungsgemäß verwendet werden. Weiterhin können Nukleotidsequenzen, Allele oder Gene verwendet werden, die sich aus der Degeneriertheit des genetischen Codes oder durch funktionsneutrale Sinnmutationen ("sense mutations") ergeben. Die Verwendung endogener Gene wird bevorzugt.

Zu den Allelen des clpB-Gens, welche funktionsneutrale Sinnmutationen enthalten, zählen unter anderem solche, die zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 40, 30, 20, 10, 5, 3 oder 2 Aminosäuren oder Aminosäurereste.

Zu den geeigneten Nukleotidsequenzen zählen auch solche, die für Proteine kodieren, in denen mindestens eine (1) Aminosäure eingefügt (Insertion) oder entfernt (Deletion) ist. Die maximale Anzahl derartige als Indels bezeichnete Veränderungen kann 2, 3, 5, 10 in keinem Falle aber mehr als 20 Aminosäuren betreffen.

Die erfindungsgemäße Nukleotidsequenz, die auch in dem erfindungsgemäßen Verfahren zum Einsatz kommt, kann durch unter anderem im Stand der Technik beschriebenen Methoden zur gerichteten Mutagenese erhalten werden.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonucleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Zur Konstruktion von Mutationen im clp-Gen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene clpB-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Die erzeugten erfindungsgemäßen Nukleotidsequenzen oder DNA-Fragmente können beispielsweise durch Transformation und/oder das Verfahren des Gen- bzw. Allelaustausches in geeignete Stämme eingebaut werden. Hierzu können insbesondere erfindungsgemäße Vektoren verwendet werden, die sich dadurch auszeichnen, dass sie die erfindungsgemäße Nukleotidsequenz oder ein DNA-Fragment, das die erfindungsgemäße Nukleotidsequenz oder Teile derselben, enthalten. Vorzugsweise enthält der Vektor zusätzlich einen Promotor. Der Vektor kann in einen geeigneten Mikroorganismus transformiert werden.

Eine gebräuchliche Methode ist die von Hamilton et al. (Journal of Bacteriology 174, 4617 - 4622 (1989)) beschriebene Methode des Genaustauschs mit Hilfe eines konditional replizierenden pSC101-Derivates pMAK705 oder mit pKO3 (Link et al., Journal of Bacteriology 179: 6228-6237). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten Nukleotidsequenzen oder Allele durch Konjugation oder Transduktion in verschiedene Stämme zu überführen.

Nähere Erläuterungen zu den Begriffen der Genetik und Molekularbiologie findet man in bekannten Lehrbüchern wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Gegenstand der Erfindung sind ferner Mikroorganismen aus der Familie der *Enterobacteriaceae,* die die erfindungsgemäße Nukleotidsequenz oder Teile dieser Nukleotidsequenz oder das erfindungsgemäße DNA-Fragment enthalten. Besagte Nukleotidsequenz liegt in exprimierter Form vor. Ebenso sind Mikroorganismen aus der Familie der *Enterobacteriaceae,* die den erfindungsgemäßen Vektor enthalten Gegenstand der Erfindung.

In einer bevorzugten Ausführungsform ist die erfindungsgemäße Nukleotidsequenz oder das erfindungsgemäße DNA-Fragment in das Chromosom integriert.

Die Mikroorganismen sind Vertreter der Familie *Enterobacteriaceae,* ausgewählt aus den Gattungen *Escherichia*, *Erwinia* und *Providencia.* Die Gattung *Escherichia* wird bevorzugt. Insbesondere ist die Art *Escherichia coli* zu nennen. Vorzugsweise wird ein Mikroorganismus verwendet, der eine erhöhte Expression des Trp-Operons aufweist.

Die erfindungsgemäßen Mikroorganismen können mit den zuvor beschriebenen Methoden zur Transformation, Transduktion oder Konjugation hergestellt werden. Vorzugsweise erfolgt die Transformation, Transduktion oder Konjugation mit dem zuvor beschriebenen erfindungsgemäßen Vektor.

Zur Herstellung der erfindungsgemäßen Stämme der Familie der *Enterobacteriaceae* werden bevorzugt Stämme verwendet (Ausgangsstämme bzw. Elternstämme), die bereits die Fähigkeit besitzen, die gewünschte aromatische L-Aminosäure, insbesondere L-Tryptophan, in der Zelle anzureichern und/oder in das sie umgebende Nährmedium auszuscheiden bzw. in der Fermentationsbrühe zu akkumulieren. Hierfür kann auch der Ausdruck "produzieren" verwendet werden. Für die Zwecke der vorliegenden Erfindung umfasst der Terminus "aromatische L-Aminosäure" die Aminosäuren L-Phenylalanin, L-Tryptophan oder L-Tyrosin. Die erfindungsgemäßen Mikroorganismen produzieren aromatischen L-Aminosäuren, vorzugsweise L-Tryptophan. Hierbei reichert sich die produzierte Aminosäure in der Zelle oder im Medium an.

Insbesondere besitzen die für die Maßnahmen der Erfindung eingesetzten Stämme die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l an aromatischen L-Aminosäuren, bevorzugt L-Tryptophan, in ≤ (maximal) 120 Stunden, ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die vorzugsweise rekombinanten Mikroorganismen mit der erfindungsgemäßen Nukleotidsequenz können aromatische L-Aminosäuren, L-Tryptophan, aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen.

Als Elternstamm geeignete, L-Tryptophan produzierende bzw. ausscheidende Stämme der Gattung *Escherichia*, insbesondere der Art *Escherichia coli* sind beispielsweise zu nennen:

| | | |
|---|---|---|
| - | Escherichia coli SV164(pGH5) | (WO 94/08031) |
| - | E. coli AGX17(pGX44) (NRRL B-12263) | (US 4,371,614) |
| - | E. coli AGX6(pGX50)aroP (NRRL B-12264) | (US 4,371,614) |
| - | Escherichia coli AGX17/pGX50,pACKG4-pps | (WO 97/08333) |
| - | Escherichia coli ATCC 31743 | (CA 1182409) |
| - | E. coli C534/pD2310,pDM136 (ATCC 39795) | (WO8 7/01130) |
| - | Escherichia coli JB102/p5LRPS2 | (US 5,939,295). |

L-Tryptophan produzierende oder ausscheidende Stämme aus der Familie der *Enterobacteriaceae* besitzen bevorzugt, unter anderem, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phosphoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Weiterhin kann es für die Produktion von aromatischen L-Aminosäuren, insbesondere L-Tryptophan, mit Stämmen der Familie *Enterobacteriaceae* vorteilhaft sein, zusätzlich ein oder mehrere Enzym(e) der bekannten Biosynthesewege oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

Darüber hinaus kann es für die Produktion von aromatischen L-Aminosäuren, insbesondere L-Tryptophan, vorteilhaft sein, zusätzlich unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die zuvor beschriebenen erfindungsgemäßen Mikroorganismen können zur Produktion von aromatischen L-Aminosäuren, vorzugsweise von L-Tryptophan, verwendet werden.

Gegenstand der Erfindung ist insbesondere ein Verfahren zur Produktion von aromatischen L-Aminosäuren oder von Futtermitteladditiven, die aromatische L-Aminosäuren enthalten, wobei ein erfindungsgemäßer Mikroorganismus in einem Medium fermentiert wird und die erhaltene L-Aminosäure in der Zelle oder im Fermentationsmedium angereichert oder aus diesem isoliert wird. Besagtes Verfahren ist insbesondere dazu geeignet, L-Tryptophan oder ein L-Tryptophan enthaltendes Futtermitteladditiv zu produzieren.

Die erfindungsgemäßen Mikroorganismen werden im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US 5,763,230) kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von L-Aminosäuren handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluss an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben. Das Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoff-haltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde. Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und d) die durch die Fermentation nicht verbrauchten Bestandteile des/ der eingesetzten Fermentationsmediums/ Fermentationsmedien bzw. der Einsatzstoffe wie beispielsweise Vitamine wie Thiamin oder Salze wie Magnesiumsulfat. Die hergestellte Kultur- bzw. Fermentationsbrühe kann anschließend gesammelt und die gewünschte L-Aminosäure bzw. das L-Aminosäure-haltige Produkt gewonnen oder isoliert werden. In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die lonenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gew.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt. Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert. Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder Granulier-Verfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von aromatischen L-Aminosäuren zur Bestimmung der Konzentration zu einem oder mehreren Zeitpunkt(en) im Verlauf der Fermentation kann durch Trennung der aromatischen L-Aminosäuren mittels lonenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur lonenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben. Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Das erfindungsgemäße Verfahren dient zur fermentativen Herstellung von organisch-chemischen Verbindungen ausgewählt aus der Gruppe der Verbindungen der aromatischen L-Aminosäuren L-Tyrosin, L-Phenylalanin und L-Tryptophan. Besonders bevorzugt ist L-Tryptophan.

### Kurze Beschreibung der Figuren

Figur 1 zeigt die Karte des das clpB-Gen enthaltenden Austauschvektors pKO3_clpBT7I.
Figur 2 zeigt die Karte des Plasmids pMU91.

Längenangaben sind als ca.-Angaben aufzufassen. Nähere Erläuterungen hierzu finden sich in den Beispielen.

### Beispiele

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America, USA (1989) 86: 2172-2175) durchgeführt.

Die Bebrütungstemperatur bei der Herstellung von Stämmen und Transformanten ist, wenn nicht anders beschrieben, 37°C.

### Beispiel 1

### Konstruktion des Austauschvektors pKO3_clpBT7I

Es konnte eine Punktmutation im clpB-Gen identifiziert werden, welche den Aminosäureaustausch an Position 7 von Threonin (T) zu Isoleucin (I) zur Folge hat.

Das clpB-Allel wird unter Anwendung der Polymerase-Kettenreaktion (PCR) sowie synthetischen Oligonukleotiden amplifiziert. Ausgehend von der Nukleotidsequenz des clpB-Gens in E. coli K12 MG1655 (Accession Number NC_000913.3, Bereich: 2731600-2734173, Blattner et al. (Science 277: 1453-1462 (1997)) werden PCR-Primer synthetisiert (Eurofins MWG GmbH, Ebersberg, Deutschland).

### Primer-Design und PCR der beiden Flanken

### Linke Flanke

| | | |
|---|---|---|
| **clpBT7I_lfw** | 5' CAGTTA**GCGGCCGC**CTGTTCGGCTCGTTCGGTAAGG 3' | |
| | random NotI | SEQ ID No.9 |
| **clpBT7I_lrv** | 5' GTTATGCGTCTGGATCGTCTTA**T**TAATAAATTCCAGCTTGCTCTTG 3' | |
| | g2732176a | SEQ ID No.10 |

Die für die PCR eingesetzte chromosomale E. coli MG1655 DNA wird nach Herstellerangaben mit "QIAGEN Genomic-tips 100/G" (QIAGEN GmbH, Hilden, Deutschland) isoliert. Mit den beiden spezifischen Primern "clpBT7I_lfw" und "clpBT7I_lrv" wird per PCR unter Standard-PCR-Bedingungen (Innis et al.: PCR Protocols. A Guide to Methods and Applications, 1990, Academic Press) mit der Pfu-DNA Polymerase (Promega Corporation, Madison, USA) mit MG1655-DNA als Template das Fragment "clpBT7I_left" amplifiziert (Länge: 539 bp). Über die Primer wird sowohl eine Notl-Restriktionsstelle, als auch die Punktmutation g2732176a in das PCR-Produkt eingeführt.

### Rechte Flanke

| | | |
|---|---|---|
| **clpBT7I_rfw** | 5' CAAGAGCAAGCTGGAATTTATTAATA**A**GACGATCCAGACGCATAAC 3' | |
| | g2732176a | SEQ ID No.11 |
| **clpBT7I_rrv** 5' TAGGTA**GCGGCCGC**CAATCGAGCGGGAACGGAAGTC 3' | | |
| | random NotI | SEQ ID No.12 |

Mit den beiden Primern "clpBT7I_rfw" und "clpBT7I_rrv" wird per PCR mit MG1655-DNA als Template das Fragment "clpBT7I_right" amplifiziert (Länge: 580 bp). Über die Primer wird sowohl eine Notl-Restriktionsstelle, als auch die Punktmutation g2732176a in das PCR-Produkt eingeführt.

### Fusion beider Flanken durch overlap-PCR

Beide Flanken werden durch eine overlap-PCR mit den beiden äußeren Primern "clpBT7I_lfw" und "clpBT7I_rrv" über ihre überlappenden Bereiche (Overlaps) miteinander fusioniert. Das resultierende Produkt "clpBT7I_Insert" hat eine Länge von 1073 bp. Nach je 6 Basen mit Zufallssequenz folgen an beiden Enden Erkennungssequenzen für das Restriktionsenzym Notl.

### Klonierung des Inserts in pKO3

Obiges Fusionsprodukt wird per Qiaquick (QIAGEN GmbH, Hilden, Deutschland) aufgereinigt und dann mit Notl verdaut. Dadurch werden Notl "sticky ends" erzeugt. Der Verdau wird erneut per Qiaquick aufgereinigt, wodurch man auch die kurzen abgeschnittenen random-Sequenzen entfernt.

Der Vektor pKO3 wird ebenfalls mit Notl geschnitten und gleichzeitig mit alkalischer Phosphatase dephosphoryliert. Anschließend wird der Verdau per Qiaquick aufgereinigt, wodurch man auch das kurze Fragment zwischen den Notl sites entfernt. Das Restriktionsprodukt besitzt ebenfalls Notl "sticky ends". Diese sind unphosphoryliert, wodurch eine Selbstligation des Plasmides verhindert wird.

Zur Ligation werden Vektor und Insert im molaren Verhältnis von 1:3 mit T4 Ligase ligiert. Chemisch kompetente Zellen des *E. coli* Klonierstammes NEB5alpha werden mit 1 µl des Ligationsansatzes transformiert und auf LB-Agar mit 20 mg/l Chloramphenicol ausgestrichen. Die Platten werden 40h bei 30°C inkubiert.

### Überprüfung der Plasmidklone

Die erfolgreiche Klonierung wird durch Spaltung des Plasmids pKO3_clpBT7I mit dem Restriktionsenzym Acul nachgewiesen.

10 Kolonien werden gepickt und über Nacht in je 10 ml LB+20 mg/l Chloramphenicol bei 30°C/180rpm kultiviert.

Am nächsten Tag werden je 2 ml der Kulturen abzentrifugiert und von den Pellets Minipreps angefertigt.

Die Ligationsprodukte können das Insert in zwei Orientierungen enthalten. Ob und in welcher Orientierung es vorliegt, lässt sich mit einem Acul-Restriktionsverdau überprüfen:
- Insert in Orientierung a: Fragmente 2872 bp und 3837 bp
- Insert in Orientierung b: Fragmente 1909 bp und 4800 bp
- pKO3 Leervektor: Fragment 5681 bp (linearisiert)

Die 10 Plasmid-Klone werden mit Acul geschnitten und die Produkte auf einem 0,8% TAE Agarosegel aufgetrennt. Ein Plasmidklon, der das Insert in Orientierung "a" enthält, wurde ausgewählt und mit "pKO3_clpBT7I" bezeichnet.

Das Insert dieses Klones wird mit den Primern "pKO3-L" und "pKO3-R" sequenziert,.

| | |
|---|---|
| **pKO3-L** 5' AGGGCAGGGTCGTTAAATAGC 3' | SEQ ID No.13 |
| **pKO3-R** 5' TTAATGCGCCGCTACAGGGCG 3' | SEQ ID No.14 |

Die DNA-Sequenz des amplifizierten Fragmentes "clpBT7I_Insert" wird unter Verwendung der Primer "pKO3-L" und "pKO3-R" bestimmt (QIAGEN GmbH, Hilden, Deutschland). Die erwartete Sequenz des clpB-Allels wurde bestätigt und das klonierte Fragment ist in SEQ ID No. 7 dargestellt. Das daraus abgeleitete partielle Genprodukt beziehungsweise Protein ist in SEQ ID No. 8 dargestellt.

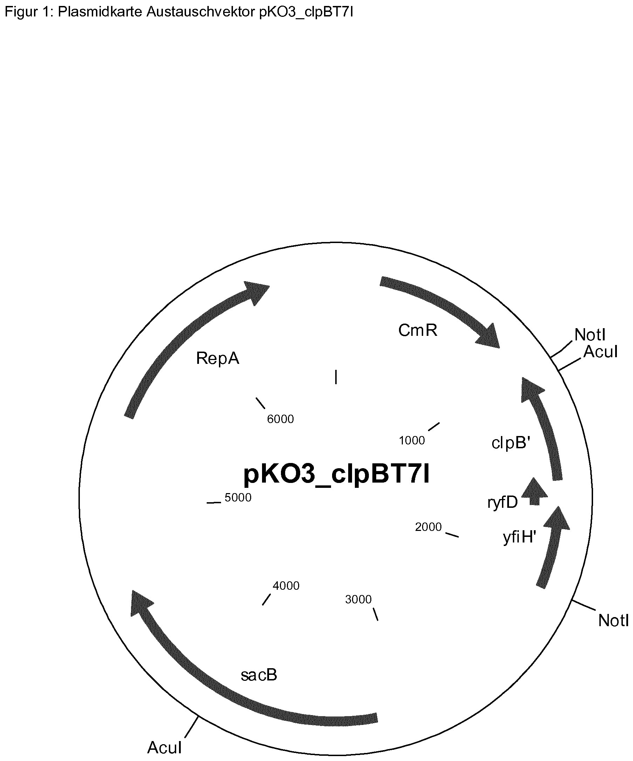

Der entstandene Austauschvektor pKO3_clpBT7I ist in Figur 1 dargestellt.

### Beispiel 2

Austausch des clpB-Wildtypgens von Stamm DM2280 gegen das clpB_T7I-Allel Ortsspezifische Mutagenese des clpB-Gens in dem E. coli Stamm DM2280/pMU91

Der L-Tryptophan produzierende E. coli Stamm Stamm DM2280/pMU91 ist ein durch P1-Transduktion erhältliches trpS⁺ Derivat des in der Patentschrift US-A-5,756,345 beschriebenen Escherichia coli K-12 Stammes JP6015/pMU91. pMU91 ist ein Plasmid abgeleitet von pSC101 (Cohen et al., Journal of Bacteriology 132: 734-737 (1977)), welches Tet^{R}, trpE476DCBA und serA⁺ trägt. JP6015/pMU91 ist hinterlegt als DSM 10123 bei dem Leibniz-Institut DSMZ-Deutschen Sammlung für Mikroorganismen und Zellkulturen (DSMZ, Braunschweig, Deutschland) in Übereinstimmung mit dem Budapester Vertrag. DM2280 ist hinterlegt als DSM 32450 bei dem Leibniz-Institut DSMZ-Deutschen Sammlung für Mikroorganismen und Zellkulturen (DSMZ, Braunschweig, Deutschland) in Übereinstimmung mit dem Budapester Vertrag.

Für den Austausch des chromosomalen clpB-Gens gegen das Plasmid-kodierte Mutationskonstrukt wurde DM2280 mit dem Plasmid pKO3_clpBT7I transformiert. Der Genaustausch erfolgt mit dem von Link et al. (Journal of Bacteriology 179: 6228-6237 (1997)) beschriebenen Selektionsverfahren und wurde durch Sequenzierung verifiziert.

Nach erfolgtem Austausch liegen in DM2280 die in SEQ ID No. 7 dargestellte Form des clpB-Allels (T7I) (Sequenzierung durch Eurofins MWG GmbH, Ebersberg, Deutschland) vor. Der erhaltene Stamm wird als DM2280_clpBT7I bezeichnet.

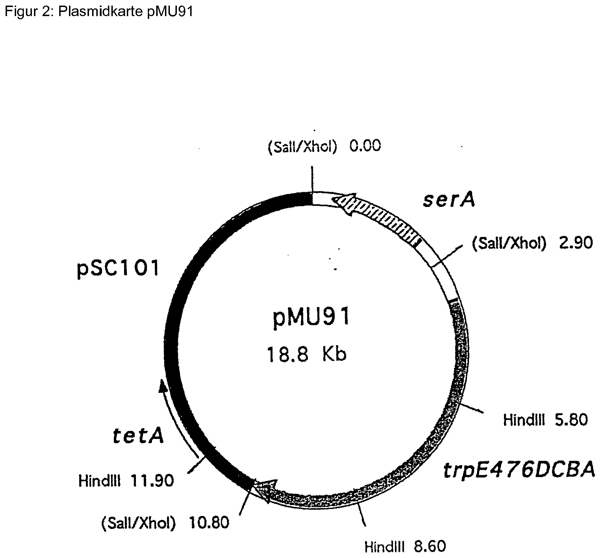

Das in der Patentschrift US-A-5,756,345 beschriebene Plasmid pMU91, welches die genetischen Informationen für die Tryptophan-Produktion trägt, wurde aus dem Stamm JP6015/pMU91 isoliert. Das Plasmid ist in Figur 2 dargestellt. Der Stamm DM2280_clpBT7I wurde mit diesem Plasmid transformiert. Einer der erhaltenen Transformanten wird als DM2280_clpBT7I/pMU91 bezeichnet.

### Beispiel 3

### Herstellung von L-Tryptophan mit dem Stamm DM2280_clpBT7I/pMU91

Die Stämme DM2280_clpBT7I/pMU91 und DM2280/pMU91 wurden auf LB-Medium mit der folgenden Zusammensetzung: 10 g/l Bacto-Trypton, 5 g/l Hefe-Extrakt, 10 g/l NaCl (pH-Einstellung auf 7,5 mit NaOH), 2 g/l Glucose, 20 g/l Agar und 5 mg/l Tetracyclin bei 30°C weiter vermehrt. Die Bildung von L-Tryptophan wird in batch Kulturen von 10 ml, die in 100 ml Erlenmeierkolben enthalten sind, überprüft. Dazu wird 10 ml Vorkulturmedium der folgenden Zusammensetzung: 1 g/l Hefeextrakt, 100 ml/l MOPS Puffer (10x), 10 g/l Glycerin und 5 mg/l Tetracyclin beimpft und für 16 Stunden bei 30°C und 150 rpm auf einem ESR Inkubator der Firma Kühner AG (Birsfelden, Schweiz) inkubiert.

10xMOPS Puffer wird aus den Lösungen A und B entsprechend den Tabellen 1 bis 3 hergestellt, zwei Volumen von Lösung A werden steril zu drei Volumen der Lösung B gegeben.

**Tabelle 1: Lösung A für 10xMOPS Puffer (sterilfiltriert).**

| Komponente | Konzentration |
|---|---|
| MOPS (Morpholinopropansulfonsäure) | 419 g/L |
| KOH (fest) | Zur Einstellung des pH-Wertes auf 7,4 |

**Tabelle 2: Lösung B für 10xMOPS Puffer. Sterilisiert durch Autoklavieren (30 Minuten, 121°C).**

| Komponente | Konzentration |
|---|---|
| Na₃ Citrat x 2H₂O | 2,35 g/l |
| FeSO₄ x 7H₂O | 0,22 g/l |
| NH₄Cl | 32,0 g/l |
| MgSO₄ x 7H₂O | 6,7 g/l |
| KCl | 4,0 g/l |
| CaCl₂ x 2H₂O | 0,25 mg/l |
| Stammlösung Spurenelemente (Tab. 4) | 3,33 ml/l |

**Tabelle 3: Stammlösung der Spurenelemente (in demin. Wasser) für 10xMOPS Puffer.**

| Komponente | Konzentration |
|---|---|
| (NH₄)₆Mo₇O₂₄ x 4H₂O | 3,7 mg/l |
| H₃BO₃ | 24,0 mg/l |
| CoCl₂ x 6H₂O | 7,1 mg/l |
| ZnSO₄ x 7H₂O | 28,7 mg/l |
| MnCl₂ x 4H₂O | 15,8 mg/l |
| CuSO₄ x 5H₂O | 2,5 mg/l |

Je 250 µl dieser Vorkultur werden in 10 ml Produktionsmedium PM1 entsprechend Tabelle 4 überimpft und für 72 Stunden bei 30°C und 150 rpm inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

**Tabelle 4: PM1 Medium: für Produktionstests in 100 ml Erlenmeierkolben**

| Komponente | Konzentration |
|---|---|
| 10xMOPS Puffer | 100 ml/l |
| Thiamine HCl (0,01%) | 1,0 ml/l |
| KH2PO4 (15mM) | 10 ml/l |
| Glucose | 10 g/l |
| Tetracyclin | 5 mg/l |

Anschließend wird die Konzentration an gebildetem L-Tryptophan im steril filtrierten Kulturüberstand durch reversed phase HPLC, so wie bei Lindroth et al. (Analytical Chemistry (1979) 51: 1167-1174) beschrieben, bestimmt.

In Tabelle 5 ist das Ergebnis des Versuches dargestellt. Der Stamm DM2280_clpBT7I/pMU91 weist gegenüber DM2280/pMU91 eine deutlich gesteigerte Produktionsleistung an L-Tryptophan auf.

**Tabelle 5: L-Tryptophan-Herstellung mit dem Stamm DM2280_clpBT7I/pMU91**

| Stamm | OD (660 nm) | L-Tryptophan g/l | Ausbeute Netto% |
|---|---|---|---|
| DM2280/pMU91 | 3,58 | 0,82 | 13,60 |
| DM2280_clpBT7I/pMU91 | 3,51 | 0,94 | 14,45 |

Die verwendeten Abkürzungen und Bezeichnungen haben folgende Bedeutung:

| | |
|---|---|
| CmR: | Chloramphenicolresistenzgen |
| sacB: | sacB-Gen aus Bacillus subtilis |
| RepA: | temperatursensitive Replikationsregion des Plasmides pSC101 |
| clpB': | Teil der Kodierregion des clpB-Gens |
| ryfD: | Kodierregion des ryfD-Gens |
| 'yfiH: | Teil der Kodierregion des yfiH-Gens |
| pSC101 : | Plasmidfragment pSC101 |
| serA : | Kodierregion des serA-Gens kodierend für die D-3-Phosphoglycerat-Dehydrogenase |
| trpE476DCBA : tetA: | Teil des Tryptophan-Operons trpLEDCBA mit trpE476-Allel Tetracyclinresistenzgen |

Die Abkürzungen für die Restriktionsenzyme haben folgende Bedeutung

| | |
|---|---|
| NotI: | Restriktionsendonuklease aus Nocardia otitidiscaviarum |
| SalI: | Restriktionsendonuklease aus Streptomyces albus G |
| HindIII: | Restriktionsendonuklease aus Haemophilus influenzae Rd |
| AcuI: | Restriktionsendonuklease aus Acinetobacter calcoaceticus |
| XhoI: | Restriktionsendonuklease aus Xanthomonas holcicola |

### In der Beschreibung aufgeführte Nicht-Patentliteratur

[1] Neidhardt (ed): Escherichia coli and Salmonella, Cellular and Molecular Biology, 2nd edition, ASM Press, Washington, D.C., USA, (1996)
[2] Blattner et al., Science 277: 1453-1462 (1997)
[3] Kitagawa et al.; Journal of Bacteriology 173(14): 4247 - 4253 (1991)
[4] T. A. Brown: Gentechnologie für Einsteiger, Spektrum Akademischer Verlag, Heidelberg, 1993
[5] Gait: Oligonucleotide synthesis: A Practical Approach (IRL Press, Oxford, UK, 1984)
[6] Newton und Graham PCR, Spektrum Akademischer Verlag, Heidelberg, 1994
[7] Horton, Molecular Biotechnology 3: 93-98 (1995): Gene Splicing by Overlap Extension
[8] Sanger et al., Proceedings of the National Academy of Science USA 74 (12): 5463-5467 (1977)
[9] Hamilton et al., Journal of Bacteriology 174, 4617 - 4622 (1989)
[10] Link et al., Journal of Bacteriology 179: 6228-6237
[11] Martinez-Morales et al., Journal of Bacteriology 1999, 7143-7148 (1999)
[12] Boyd et al. (Journal of Bacteriology 182, 842-847 (2000))
[13] Birge: Bacterial and Bacteriophage Genetics, 4th ed., Springer Verlag, New York (USA), 2000
[14] Berg, Tymoczko and Stryer: Biochemistry, 5th ed., Freeman and Company, New York (USA), 2002
[15] Sambrook et al.: Molekular Cloning, A Laboratory Manual, (3-Volume Set), Cold Spring Harbor Laboratory Press, Cold Spring Harbor (USA), 2001
[16] Nakayama: Breeding of Amino Acid Producing Microorganisms, in: Overproduction of Microbial Products, Krumphanzl, Sikyta, Vanek (eds.), Academic Press, London, UK, 1982
[17] Chmiel: Bioprozesstechnik 1. Einführung in die Bioverfahrenstechnik (Gustav Fischer Verlag, Stuttgart, 1991)
[18] Storhas: Bioreaktoren und periphere Einrichtungen (Vieweg Verlag, Braunschweig/Wiesbaden, 1994)
[19] Manual of Methods for General Bacteriology, American Society for Bacteriology (Washington D.C., USA, 1981)
[20] Spackman et al., Analytical Chemistry 30: 1190-1206 (1958)
[21] Pickering, LC·GC (Magazine of Chromatographic Science) 7(6), 484-487 (1989)
[22] Lindroth et al.,Analytical Chemistry 51: 1167-1174 (1979)
[23] Lottspeich und Zorbas: Bioanalytik (Spektrum Akademischer Verlag, Heidelberg, Deutschland 1998).
[24] J.H. Miller: A Short Course in Bacterial Genetics (1992), Cold Spring Harbor Laboratory Press
[25] Sambrook et al.: Molecular Cloning - A Laboratory Manual (1989), Cold Spring Harbor Laboratory Press
[26] Chung et al. (Proceedings of the National Academy of Sciences of the United States of America, USA (1989) 86: 2172-2175
[27] Innis et al.: PCR Protocols. A Guide to Methods and Applications, 1990, Academic Press
[28] Link et al., Journal of Bacteriology 179: 6228-6237 (1997)

## Patentansprüche

1. Nukleotidsequenz, die für eine Variante des Hitzeschockproteins CIpB kodiert, und deren Aminosäuresequenz zu mindestens 95 % identisch ist mit SEQ ID NO:2, **dadurch gekennzeichnet, dass** in der Aminosäuresequenz der L-Threoninrest an Position 7 gegen einen L-Isoleucinrest ersetzt ist.

2. Nukleotidsequenz nach Anspruch 1, **dadurch gekennzeichnet, dass** das kodierte Polypeptid im Wesentlichen eine Länge entsprechend 857 Aminosäuren aufweist.

3. Nukleotidsequenz, **dadurch gekennzeichnet, dass** sie die Nukleotidsequenz gemäß einem der Ansprüche 1 oder 2 enthält oder ein Fragment der Nukleotidsequenz gemäß einem der Ansprüche 1 oder 2 darstellt, wobei ein solches Fragment mindestens für die Positionen 5 bis 820 der Aminosäuresequenz gemäß Anspruch 1 kodiert.

4. Vektor, **dadurch gekennzeichnet, dass** er die Nukleotidsequenz gemäß einem der Ansprüche 1 bis 3, sowie gegebenenfalls einen Promotor enthält.

5. Mikroorganismus aus der Familie der *Enterobacteriaceae,* **dadurch gekennzeichnet, dass** er die Nukleotidsequenz gemäß einem der Ansprüche 1 bis 3 enthält.

6. Mikroorganismus aus der Familie der *Enterobacteriaceae,* **dadurch gekennzeichnet, dass** er den Vektor gemäß Anspruch 4 enthält.

7. Mikroorganismus aus der Familie der *Enterobacteriaceae,* **dadurch gekennzeichnet, dass** die Nukleotidsequenz gemäß einem der Ansprüche 1 bis 3 in das Chromosom integriert ist.

8. Mikroorganismus gemäß einem der Ansprüche 5 bis 7, **dadurch gekennzeichnet, dass** er ausgewählt ist aus den Gattungen *Escherichia*, *Erwina*, und *Providencia.*

9. Mikroorganismus gemäß einem der Ansprüche 5 bis 8, **dadurch gekennzeichnet, dass** dieser organisch-chemische Verbindungen ausgewählt aus der Gruppe der aromatischen L-Aminosäuren produziert.

10. Mikroorganismus gemäß Anspruch 9, **dadurch gekennzeichnet, dass** es sich bei der aromatischen L-Aminosäure um L-Tryptophan handelt.

11. Mikroorganismus gemäß einem der Ansprüche 5 bis 10, **dadurch gekennzeichnet, dass** der Mikroorganismus eine erhöhte Expression des Trp-Operons aufweist.

12. Verfahren zur Herstellung eines Mikroorganismus gemäß einem der Ansprüche 5 bis 11 durch Transformation, Transduktion oder Konjugation, **dadurch gekennzeichnet, dass** die Transformation, Transduktion oder Konjugation mit einem Vektor gemäß Anspruch 4 erfolgt.

13. Verwendung eines Mikroorganismus gemäß einem der Ansprüche 5 bis 11 zur Produktion von L-Tryptophan.

14. Verfahren zur Produktion von aromatischen L-Aminosäuren oder von Futtermitteladditiven, die aromatische L-Aminosäuren enthalten, **dadurch gekennzeichnet, dass** es die Fermentation eines Mikroorganismus nach einem der Ansprüche 5 bis 11 in einem Medium sowie das Anreichern der aromatischen L-Aminosäure im Fermentationsmedium und/oder das Isolieren der aromatischen L-Aminosäure umfasst.

15. Verfahren gemäß Anspruch 14, **dadurch gekennzeichnet, dass** es sich bei der aromatischen L-Aminosäure um L-Tryptophan handelt.

## Claims

1. Nucleotide sequence which encodes a variant of the heat shock protein ClpB, and the amino acid sequence of which is at least 95% identical to SEQ ID NO:2, **characterized in that** the L-threonine residue at position 7 in the amino acid sequence has been replaced by an L-isoleucine residue.

2. Nucleotide sequence according to Claim 1, **characterized in that** the encoded polypeptide has substantially a length corresponding to 857 amino acids.

3. Nucleotide sequence, **characterized in that** it contains the nucleotide sequence according to either of Claims 1 and 2 or is a fragment of the nucleotide sequence according to either of Claims 1 and 2, such a fragment encoding at least positions 5 to 820 of the amino acid sequence according to Claim 1.

4. Vector, **characterized in that** it contains the nucleotide sequence according to any of Claims 1 to 3 with or without a promoter.

5. Microorganism from the *Enterobacteriaceae* family, **characterized in that** it contains the nucleotide sequence according to any of Claims 1 to 3.

6. Microorganism from the *Enterobacteriaceae* family, **characterized in that** it contains the vector according to Claim 4.

7. Microorganism from the *Enterobacteriaceae* family, **characterized in that** the nucleotide sequence according to any of Claims 1 to 3 is integrated into the chromosome.

8. Microorganism according to any of Claims 5 to 7, **characterized in that** it is selected from the genera *Escherichia*, *Erwina*, and *Providencia.*

9. Microorganism according to any of Claims 5 to 8, **characterized in that** it produces organic chemical compounds selected from the group of the aromatic L-amino acids.

10. Microorganism according to Claim 9, **characterized in that** the aromatic L-amino acid is L-tryptophan.

11. Microorganism according to any of Claims 5 to 10, **characterized in that** the microorganism has an increased expression of the Trp operon.

12. Method for producing a microorganism according to any of Claims 5 to 11 by transformation, transduction or conjugation, **characterized in that** the transformation, transduction or conjugation is done using a vector according to Claim 4.

13. Use of a microorganism according to any of Claims 5 to 11 for producing L-tryptophan.

14. Method for producing aromatic L-amino acids or feedstuff additives containing aromatic L-amino acids, **characterized in that** it comprises the fermentation of a microorganism according to any of Claims 5 to 11 in a medium and also the enrichment of the aromatic L-amino acid in the fermentation medium and/or the isolation of the aromatic L-amino acid.

15. Method according to Claim 14, **characterized in that** the aromatic L-amino acid is L-tryptophan.

## Revendications

1. Séquence nucléotidique, qui code pour une variante de la protéine de choc thermique ClpB, et dont la séquence d'acides aminés est au moins 95 % identique à SEQ ID NO : 2, **caractérisée en ce que,** dans la séquence d'acides aminés, le radical L-thréonine à la position 7 est remplacé par un radical L-isoleucine.

2. Séquence nucléotidique selon la revendication 1, **caractérisée en ce que** le polypeptide codé présente essentiellement une longueur correspondant à 857 acides aminés.

3. Séquence nucléotidique, **caractérisée en ce qu'**elle contient la séquence nucléotidique selon l'une quelconque des revendications 1 ou 2 ou est un fragment de la séquence nucléotidique selon l'une quelconque des revendications 1 ou 2, un tel fragment codant au moins pour les positions 5 à 820 de la séquence d'acides aminés selon la revendication 1.

4. Vecteur, **caractérisé en ce qu'**il contient la séquence nucléotidique selon l'une quelconque des revendications 1 à 3, ainsi qu'éventuellement un promoteur.

5. Microorganisme de la famille des *Enterobacteriaceae,* **caractérisé en ce qu'**il contient la séquence nucléotidique selon l'une quelconque des revendications 1 à 3.

6. Microorganisme de la famille des *Enterobacteriaceae,* **caractérisé en ce qu'**il contient le vecteur selon la revendication 4.

7. Microorganisme de la famille des *Enterobacteriaceae,* **caractérisé en ce que** la séquence nucléotidique selon l'une quelconque des revendications 1 à 3 est intégrée dans le chromosome.

8. Microorganisme selon l'une quelconque des revendications 5 à 7, **caractérisé en ce qu'**il est choisi parmi les genres *Escherichia*, *Erwina* et *Providencia.*

9. Microorganisme selon l'une quelconque des revendications 5 à 8, **caractérisé en ce que** celui-ci produit des composés chimiques organiques choisis dans le groupe des acides L-aminés aromatiques.

10. Microorganisme selon la revendication 9, **caractérisé en ce que** l'acide L-aminé aromatique est le L-tryptophane.

11. Microorganisme selon l'une quelconque des revendications 5 à 10, **caractérisé en ce que** le microorganisme présente une expression augmentée de l'opéron Trp.

12. Procédé de fabrication d'un microorganisme selon l'une quelconque des revendications 5 à 11 par transformation, transduction ou conjugaison, **caractérisé en ce que** la transformation, la transduction ou la conjugaison a lieu avec un vecteur selon la revendication 4.

13. Utilisation d'un microorganisme selon l'une quelconque des revendications 5 à 11 pour la production de L-tryptophane.

14. Procédé de production d'acides L-aminés aromatiques ou d'additifs d'aliments pour animaux, qui contiennent des acides L-aminés aromatiques, **caractérisé en ce qu'**il comprend la fermentation d'un microorganisme selon l'une quelconque des revendications 5 à 11 dans un milieu, ainsi que l'enrichissement de l'acide L-aminé aromatique dans le milieu de fermentation et/ou l'isolement de l'acide L-aminé aromatique.

15. Procédé selon la revendication 14, **caractérisé en ce que** l'acide L-aminé aromatique est le L-tryptophane.
